# EUROPEAN PATENT SPECIFICATION

(11) **EP 4 195 219 B1**
(45) Date of publication and mention of the grant of the patent: **29.07.2026**
(21) Application number: 21213382.1
(22) Date of filing: 09.12.2021
(51) Int. Cl.: G16B 25/00, G16B 40/10, G16B 40/20, G16H 50/30

(54) **MEANS AND METHODS FOR THE BINARY CLASSIFICATION OF MS1 MAPS AND THE RECOGNITION OF DISCRIMINATIVE FEATURES IN PROTEOMES**
MITTEL UND VERFAHREN ZUR BINÄREN KLASSIFIZIERUNG VON MS1-KARTEN UND ZUR ERKENNUNG VON UNTERSCHEIDUNGSMERKMALEN IN PROTEOMEN
MOYENS ET PROCÉDÉS DE CLASSIFICATION BINAIRE DE CARTES MS1 BINAIRE ET DE RECONNAISSANCE DE CARACTÉRISTIQUES DISCRIMINANTES DANS DES PROTÉOMES

(43) Date of publication of application: 14.06.2023
(73) Proprietor: MOLEQLAR Analytics GmbH, 81671 München (DE)
(72) Inventor: Solis, Victor, 82152 Planegg (DE); Paul, Soumya, 82152 Planegg (DE)
(74) Representative: Schiweck Weinzierl Koch Patentanwälte Partnerschaft mbB

(56) References cited:
- SHISHENG WANG ET AL: "MSpectraAI: a powerful platform for deciphering proteome profiling of multi-tumor mass spectrometry data by using deep neural networks", BMC BIOINFORMATICS, BIOMED CENTRAL LTD, LONDON, UK, vol. 21, no. 1, 7 October 2020 (2020-10-07), pages 1 - 15, XP021282553, DOI: 10.1186/S12859-020-03783-0
- ZHANG FANGFEI ET AL: "Phenotype Classification using Proteome Data in a Data-Independent Acquisition Tensor Format", JOURNAL OF THE AMERICAN SOCIETY FOR MASS SPECTROMETRY, vol. 31, no. 11, 26 October 2020 (2020-10-26), US, pages 2296 - 2304, XP055917599, ISSN: 1044-0305, DOI: 10.1021/jasms.0c00254
- CADOW JORIS ET AL: "On the feasibility of deep learning applications using raw mass spectrometry data", vol. 37, no. Supplement_1, 4 August 2021 (2021-08-04), GB, pages i245 - i253, XP055916683, ISSN: 1367-4803, Retrieved from the Internet <URL:https://watermark.silverchair.com/btab311.pdf?token=AQECAHi208BE49Ooan9kkhW_Ercy7Dm3ZL_9Cf3qfKAc485ysgAAAwwwggMIBgkqhkiG9w0BBwagggL5MIIC9QIBADCCAu4GCSqGSIb3DQEHATAeBglghkgBZQMEAS4wEQQMHt-Vf-Ej3rc9kI0wAgEQgIICvyrWQk-HxHaTWF40pnco0NxT2n3hWX_qjZKF8YojcBRif01_jpTLau-WXTjZnMTgHi20delYTzrBEf3UTq9X7FYTYC80> DOI: 10.1093/bioinformatics/btab311
- "Medical Image Computing and Computer Assisted Intervention", 29 September 2020, SPRINGER, CHAM, ISBN: 978-3-030-58594-5, article CHENG YUAN, YUJIN TANG, DAHONG QIAN: "Ovarian Cancer Prediction in Proteomic Data Using Stacked Asymmetric Convolution", pages: 263 - 271, XP047587314, DOI: https://doi.org/10.1007/978-3-030-59713-9_26

## Description

Mass spectrometry (MS)-based proteomics has become a powerful technology for the identification, characterization, and quantification of proteins from cell cultures, organells and tissues, thereby identifying abundance levels, modifications, and interactions (Aebersold & Mann 2003, "Mass spectrometry-based proteomics, Nature 422: 198-207; Angel et al. 2012, "Mass spectrometry based proteomics: existing capabilities and future directions, Chem Soc Rev 41(10): 3912-3928). While the proteome is highly complementary to the genome, it differs from cell-to-cell and time-to-time and should be profoundly descriptive of biological phenotype.

Proteomics is essential for deciphering how molecules interact as a system and for understanding the functions of cellular systems in human disease; however, the unique characteristics of the human proteome, which include a high dynamic range of protein expression and extreme complexity due to a plethora of post-translational modifications (PTMs) and sequence variations, make such analyses challenging (Gregorich & Ge 2014, "Top-down Proteomics in Health and Disease: Challenges and Opportunities", Proteomics 14(10): 1195-1210).

MS-based proteomics is unbiased in the sense that it identifies and quantifies proteins in an untargeted manner. Additionally, the identification is extremely specific through the amino acid sequence information at the peptide level. These inherent features differentiate MS-based from affinity-based methods and should make MS an ideal tool for biomarker discovery.

However, discovering biomarkers in proteomes is currently limited by the traditional use of only identified (ID-ed) signals, i.e., signals labeled with a specific peptide/protein ID. Because of this traditional approach, many signals remain unused during discovery. Furthermore, most ID-driven models condensed the interrelations between signals (i.e. peptide/protein intensities) with linear models that only exploit the ID-ed signals and their quantities, but not other properties present only on raw unprocessed data. Another challenge is the normalization and integration of data coming from different experimental batches, biological materials or instruments and LC-MS separation/acquisition methods. Finally, an important challenge is to build interpretability of the data, so that it ensures trust in the final application by scientists and doctors.

Thus, further means and methods are needed that may improve the biased discovery approach of current methods which ignore un-ID-ed signals, i.e. novel methods that, in principle, allow the recognition of the entirety of discriminative features in MS-based proteomics. For example, it is desired to find distinctive features (presence, absence or differentially abundant) in MS spectra (maps) as potential markers of a condition/disease of a subject. Specifically, there is a need for providing means and methods which exploit specific properties of MS spectra from individuals of different health states, thereby using additional information from a MS spectra such as elution time of the signal, shape of the signal and other properties present only on raw unprocessed MS data. Hence, these means and methods ideally tell whether a MS spectrum belongs to a diseased versus a healthy individual or a treated versus an untreated subject/specimen or, in general, to a clinical/biological condition A versus a condition B or C. Moreover, the method will tell the peptide features which led the means and methods to that decision. Wang et al. "MSpectraAI: a powerful platform for deciphering proteome profiling of multi-tumor mass spectrometry data by using deep neural networks", DOI: 10.1186/S12859-020-03783-0 discloses a method for classifying tumour types from MS images that represent patient proteomes, by iteratively training a neural network on the same dataset.

Hence, the present invention addresses these needs and provides as solution thereto the means and methods described hereinafter, defined in the claims, illustrated in the examples and shown in the figures.

### SUMMARY OF THE INVENTION

The invention is set out in the appended claims.

### SUMMARY OF RELATED DISCLOSURE

Accordingly the present invention provides a computer-implemented method for predicting a condition of health of a patient to be examined from a mass spectrometry, MS, specimen image representing a proteome of said patient, wherein x and y coordinates of a picture element, pixel, of the image indicate mass and elution time, also known as retention time, of a molecule in the proteome, and a value of the pixel indicates an abundance of the molecule in the proteome, the method comprising:
first training a neural network, NN, via an input layer, with a first plurality of sample MS images, also referred to as MS1 images, each sample image representing a proteome fulfilling a known first condition from a set of known conditions,
subsequent second training the NN, via the input layer, with a second plurality of sample MS1 images from a plurality of patients, each sample image representing a proteome fulfilling a known second binary (or multiclass) condition of health from a plurality of patient conditions,
wherein the first training of the NN facilitates the subsequent second training of the NN;
obtaining, after the second training of the NN, in an input layer of the NN, a mass spectrometry, MS, image, representing a proteome expressing a condition of health of a patient to be examined;
forward propagating the MS image through the NN by repeatedly comparing outputs of the NN with corresponding ground truth labels, and applying a loss function to the resulting error to adjust weights and biases of the NN;
generating, via an output layer of the NN, a prediction of a condition of health of the patient to be examined .

"Forward propagation" is used to obtain neuron activations in consecutive layers of a plurality of layers of the NN from one or more input variables, yielding one or more output variables of the NN.

A "loss function" defines a measure of an error between one or more output variables of the NN and corresponding one or more ground truth labels.

A "ground truth label" of the NN represents a target (expected output) variable corresponding to a respective input variable of the NN.

By updating, known as "back-propagation", the current weights and biases of each layer of the plurality of layers of the network, based on the derivative of the "loss function" and the measure of the current error, the weights and biases can be improved for repeated forward propagations, thereby minimizing the "loss function".

The iterative procedure of "forward-propagation" and "back-propagation" while aiming to minimizing the "loss function" entails the training of the NN.

In (Loy Neural _Network Projects with Python (Packt Publishing, 2019) a simple example of training a neural network is presented, for instance.

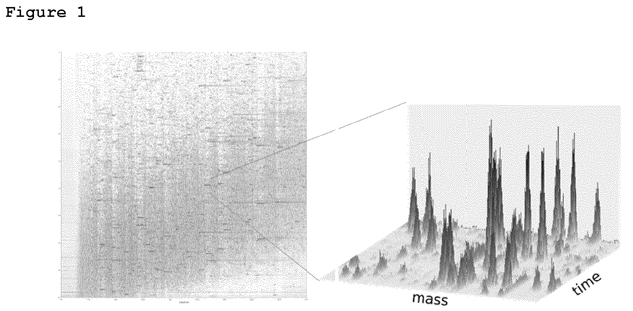

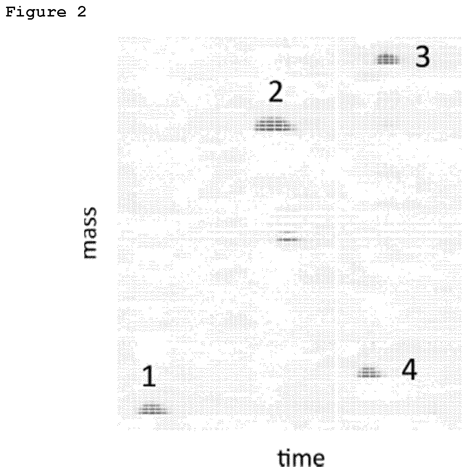

The computer-implemented methods of the present invention, hereinafter also simply referred to as "method" or "methods" of the present invention, make use of a NN for image classification that distinguishes between MS spectra from diseased people and heathy individuals. Specifically, said MS spectra is a 2D representation of the proteome of an individual, wherein the Y axis indicates the mass and the X axis the elution time of a molecule, and a value of a pixel reflects the abundance of the molecule (see **Figures 1** and **2**). Said 2D heatmap-like images are called MS1 maps throughout this application. Furthermore, the methods of the present invention specifically aim at finding distinctive un-ID-ed signals in said MS1 maps, i.e. a model interpreter is employed to point out these distinctive signals on the MS1 map in order to further characterize them later.

In this respect raw MS data stored in public databases, such as PRIDE, or in proprietary databases can be utilized during training of the NN. If acquiring a cohort with enough individuals per class is impractical the obtained individuals can be measured 5 times each, which will lead to data augmentation per se. Another option is to use generative adversarial networks (GANs) trained first in MS1 maps of any class and then further train it using only MS1 maps of the two classes of interest. The classes will be binary in most of the training problems; e.g.: SILAC versus label-free; diseased vs. healthy; etc. Nonetheless, multiclass problems/conditions are also considered; e.g.: tissue A versus tissue B versus tissue C; disease stage 1 versus disease stage 2 versus disease stage 3, and are described elsewhere herein. The model of the present invention focuses on proteomics data.

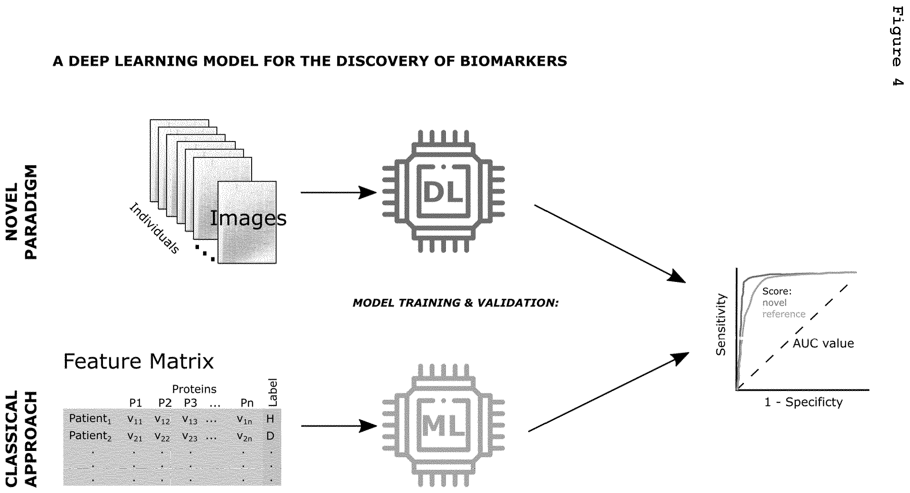

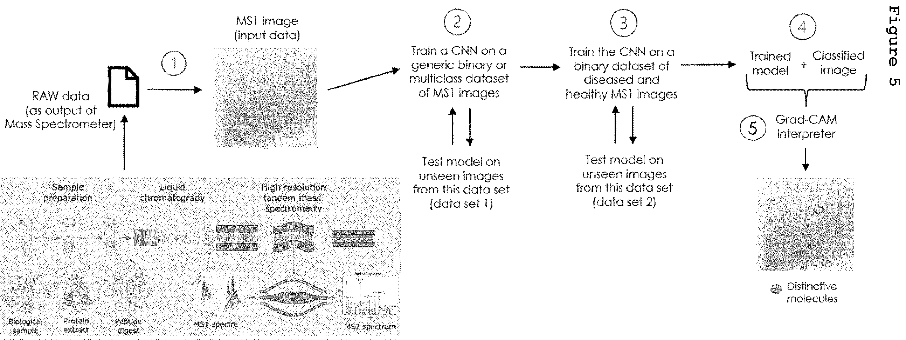

The so called "Deep Learning (DL)" modelling approach of the present invention versus the classical "Machine Learning (ML)" approach that uses protein abundance matrices is depicted in **Figure 4****.** The workflow of the AI platform for the binary classification of MS1 maps and the recognition of discriminative features according to the present invention is summarized in **Figure 5****.**

Methods of Deep Learning using *artificial neural networks* are described in a textbook by Goodfellow, I., Bengio, Y. & Courville, A. Deep Learning (MIT Press, 2016), for instance. However, the application for predicting conditions of health of a patient using MS-based proteomic images has never been described so far.

The whole platform used according to the method of the present invention is explained with more detail in the following and typically entails
(i) a data converter that transforms RAW/mzXML files to PNG/JPEG images (or other appropriate format),
(ii) a prebuilt model obtained from ImageNet convolutional neural network (CNN)/ResNet models and trained, for instance, in SILAC vs Label-free data
(iii) a final CNN/ResNet classifier obtained from the above prebuilt model, further trained, for instance, in breast cancer versus healthy MS1 images
(iv) a Saliency Map tool (also refer as the "interpreter"), telling the MS1 molecular features that the NN image classifier used for making its decision, and
(v) a web application, wrapping all components above into a graphical user interface (GUI).

The data converter tool (i) can be an app/module that translates RAW/mzXML/mzML spectra into 2D images in PNG/JPEG or a better suited format. The image depicts the MS1 map in high enough resolution as to distinguish peptide ion features in the map. The image displays the data as a heatmap, where the Y-axis indicates the mass of the peptide ion and the X-axis displays the elution time of the peptide ion. The signal intensity in the 2D map is indicated with heatmap pixels that reflect the abundance of the molecule in the subject/specimen. The data converter tool must allow the following extraction parameters: selection of m/z range and elution time range, selection of final image resolution (ppi) and optionally a signal intensity normalization function. The front-end of this tool, should accept the raw file (or a file containing the list of raw files) and selection buttons to define the parameters above. The output shall be one image per raw file.

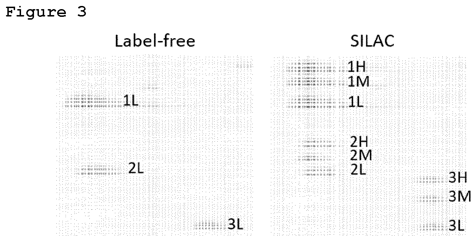

The prebuilt CNN/ResNet model (ii) is trained as follows. The creation and training will start with a toy case where the classifier will need to discriminate between SILAC and Label-free MS1 maps. This kind of data is a good training set because SILAC MS1 maps contain distinctive signals that are absent in Label-free MS1 maps (see **Figure 3****).** An analogous situation will be found when dealing with clinical data; e.g. some molecules will be present only in cancer samples and some others only in healthy samples. Finding the distinctive signals in SILAC versus Label-free maps is fairly easy for a human and, therefore, shall allow the validation of the CNN/ResNet model. If the model is learning well, those distinctive signals (i.e., features) should be picked by the model in order to classify the two groups. Sensitivity and Specificity in this task must be above 95%, as this threshold is feasible based on previous trainings and tests. The outcome of this phase is a prebuilt AI model that is able to classify MS1 images into the two conditions. This model serves as the basis to obtain a final model that classifies images coming from more specific, case-control clinical cohorts.

The final CNN model (iii), i.e. the trained, tested and validated image classifier from step (ii) is, for example, trained with MS1 images from cancer (N=250-500) versus healthy subjects (N=250-500) with the aim to obtain an accuracy of >90% (sensitivity >85% and specificity >95%) in the test set. Labeling of the MS1 images is straightforward as it will be known which come from cancerous samples and which from healthy samples. The idea behind starting with a pre-trained model is to use the intermediate representations (peptide features) that the model has learned from the generic and less scarce MS1 images from prebuilt CNN/ResNet model (ii), to then further train the classification layers of the CNN model with the scarcer cancer and healthy images and solve, thereby, the actual clinical classification problem in hand. After this second training, prediction accuracy shall be further improved by fine-tuning the weights from the top layers of the prebuilt model. All training and fine-tuning can be done e.g. using the software library Tensorflow.

All in all, the final CNN model will perform, in the end, better than traditional approaches for biomarker discovery (see **Figure** 4). This better performance is expected due to i) the use of all peptide signals in the data versus only ID-ed ones and ii) due to the use of Deep Learning models, which catch more complexity and interrelations in the data compared to classical machine learning (ML) approaches. Regarding the first reason, classical approaches of biomarker discovery in proteomics start by identifying the peptide sequence IDs of the signals in the MS1 map. However, due to the lack of scientific knowledge about protein isoforms and post-translational modifications, only 30-50% of the signals in a MS1 map get ID-ed; the rest remain unused. In the approach of the present invention, all signals in the MS1 map are fed to the NN model, which shall use the distinctive signals between the two classes, by either presence/absence of the signal or by differential intensities.

The final CNN model (iii) will be subject to a Saliency Map tool interpretation (iv), i.e. a method of Saliency Map explanation (e.g., Gradient-weighted Class Activation Mapping: Grad-CAM) to explain the decision of the NN model. Besides Grad-CAM interpretation (as disclosed in Selvaraju et al. 2019, "Grad-CAM: Visual Explanations from Deep Networks via Gradient-based Localization", arXiv:1610.02391), also other interpreters such as Guided Backpropagation, Guided Grad-CAM, Vainilla Gradient, SmoothGrad, SmoothGrad-Grad-CAM can be applied in the context of the present invention.

In other words, the interpreter highlights which features (and regions) in an MS1 map were the most decisive for the CNN model to classify an image as, for example, diseased or healthy. The interpreter receives the CNN model, an image (i.e., an MS1 map) and the predicted class label (i.e., the one given by the NN image classifier). Next, it forward propagates the image through the NN while setting the gradients for the other classes to 0. The signal obtained from the last NN layer is then back-propagated to the rectified convolutional feature maps of interest, which are finally combined to compute a coarse localization heatmap on top of the image. Grad-CAM, in particular, is applicable as a modification of a wide variety of CNN model-architectures.

The AI platform described above is provided within a web/computer/mobile application (v), i.e. the AI platform will be run in the back-end of a Graphical User Interface (GUI) where scientists and doctors can provide a MS1 map of a patient and received as output an annotated MS1 image with the distinctive peptides highlighted and (if possible) ID-ed. For the identification of peptides, a so called peptide spectrum match (PSM) is performed between the observed fragment spectrum of a peptide, i.e. the MS2 spectrum, and the theoretical fragment spectra of a number of peptide candidates that have almost the same molecular mass. The candidate with the best PSM score is then chosen as the ID of the peptide MS1 signal. The theoretical fragment spectra of the candidates can e.g. be obtained by i) in-silico fragmentation of a peptide sequence (R.G.Sadygov et al. 2004, Nature Methods, 1,3, pp 195-202; Perkins et al. 1999, Electrophoresis, 20, 3551-3567), ii) a collection of previously identified MS2 spectral libraries (Yates et al. 1998, Anal Chem., 70(17):3557-65; Lam et al. 2008, Nat Methods, 5(10):873-5) or iii) in-silico generation of spectral libraries (Röst 2019, Nat Methods, 16(6):469-470; Schmidt et al. 2019, Nat Methods, 16(6):509-518). When such a collection of theoretical spectra is not available, a so called de-novo approach is employed. This de-novo approach infers the sequence of the MS1 peptide feature based on the mass separation of the fragment masses recorded in its MS2 spectrum. A final, but not less important case happens when the MS1 peptide feature is observed, but the mass spectrometer did not generate a MS2 spectrum for that peptide or it did, but the degree of fragmentation in this spectrum was too poor to obtain a confident PSM. In those cases, the experimenter goes back to the laboratory and performs a MS targeted acquisition of MS2 data for all those MS1 peptide features and then uses a combination of de-novo and PSM methods to infer the ID of the peptide features.

In sum, the method of the present invention is based on the recognition of the entirety of discriminating features of a proteome, and not only on ID-driven peptides, analyzed by mass spectrometry, MS. In particular, the present inventors found that using the full information/signals of MS raw data allows them to recognize the entirety of discriminative features in MS-based proteomics. The MS specimen image generated from the MS raw data is then used for predicting a condition of health of a patient to be examined. In this respect, the neural network is trained so that a classification of a condition of health can be achieved in a more accurate way. The means and methods of the present invention bring thus benefit to the field of predicting or diagnosing a condition of health.

In the context of the present invention, the term "mass spectrometry, MS, specimen image" can interchangeably be used with the term "MS1 map". Said MS1 map refers to a 2D representation (2D heatmap-like images) of the proteome of an individual, wherein the Y axis indicates the mass and the X axis the elution time of a molecule, and a value of a pixel reflects the abundance of the molecule (see **Figures 1** and **2**). As described elsewhere herein, said MS1 maps are obtained from MS raw data, which are translated by a data converter tool into MS1 maps.

The MS raw data used and converted in the context of the present invention into MS1 maps are obtainable by various mass spectrometry techniques, e.g. Gas Chromatography Mass Spectrometry (GC-MS) or Liquid Chromatography Mass Spectrometry (LC-MS) which are known to those skilled in the art. In the context of the present invention, MS raw data converted into MS1 maps are preferably obtained by LC-MS.

The term "predicting a condition of health" as used in the context of the present invention relates to the likelihood that a certain condition of health is present in the patient to be examined from a MS specimen image, i.e. it prognosticates or forecasts a certain health condition in said patient. A "condition of health" or "health condition" when recited herein is understood as a medical or physical status of said patient to be examined. Said health condition can be a healthy condition or a disease state of said patient. Hence, "predicting a condition of health" according to the method of the present invention means that said method is finally used to assess whether said patient is healthy or diseased. Hence, the method of the present invention is also used for diagnosing a disease. Further, the present invention can also be used to predict a biological condition of a specimen, comprising the steps of the method for predicting a condition of health defined herein. In this respect, a biological condition of a specimen refers to any phenotype not necessarily associated with a disease, as for example, cell stage of differentiation, cell or tissue type, or the source organism, where a specimen can be any kind of sample taken from a subject as defined elsewhere herein.

The term "patient" when used herein refers to a subject or individual - "patient" may be used interchangeably with "subject" or "individual" and vice versa - that makes use of medical services or to whom medical services are provided. However, the term "patient" does not mean that said subject is already known to suffer from a certain disease. Instead, the provided method of predicting a condition of health can finally provide that said patient is in a healthy condition, or otherwise reveal that said patient suffers from a certain disease. Certainly, the method of the present invention can also be used to confirm that a patient suffers from a certain disease. Alternatively, the method of predicting a health condition with the present invention can also be used to monitor the course of a certain disease, thereby providing information on whether the disease mitigates under therapeutic measures.

The "patient" in the context of the present invention is preferably a mammal. The mammal may be any one of mouse, rat, guinea pig, rabbit, cat, dog, monkey, horse, or human. Preferably, the "patient" in the context of the present invention is a human patient.

The term "proteome" as used in the context of the present invention refers to the entire set of proteins that is, or can be, expressed by a genome, cell, tissue, or organism at a certain time. It is the set of expressed proteins in a given type of cell or organism, at a given time, under defined conditions.

"Proteomics" is the study of the proteome. As mentioned above, the proteome of an organism differs from cell-to-cell and time-to-time and can be used to determine a condition of health of a patient based on certain molecules/biomarkers in said proteome.

In the context of the present invention, the proteome to be analyzed by MS technology can derive from any kind of patient sample that contains proteins, such as a saliva sample, a serum sample, a tissue sample, a blood sample, a urine sample, a lymphatic fluid sample, a nasopharyngeal wash sample, a sputum sample, a mouth swab sample, a throat swab sample, a nasal swab sample, a bronchoalveolar lavage sample, or a bronchial secretion sample, just to name some. The proteome is extracted from said samples using known proteome extraction methods, which are based for example on acid extraction (Doellinger et al. 2020, Technological Innovation and Resources, Vol. 19(1): 209-222). In view to the patients to be examined and the condition of health to be evaluated, the appropriate sample will be selected and processed for proteome analysis by those skilled in the art.

In a specific embodiment of the method of the present invention, it is envisaged that the proteome to be analyzed exclusively comprises, preferably consists of histones extracted from a certain sample, i.e. the sample from a patient to be examined is processed to contain histones only. Said histone sample will then be used to generate MS raw data that are further converted into MS1 maps as described elsewhere herein. Hence, the method of the present invention also refers to the prediction of a condition of health of a patient to be examined from MS images representing the entirety of histone information of said patient. Both differential histone levels and differential histone modifications can be detected by the method. Because histone levels and their modifications are associated with certain diseases, our platform will lead to the discovery of epigenetic biomarkers in, for example cancer, obesity, cardiovascular diseases, diabetes as well as epigenetic biomarkers associated with sport fitness and healthy diets.

As described elsewhere herein, the method of predicting a condition of health of a patient to be examined from MS specimen image comprises the step of a first training of a neural network, NN, via an input layer, with a first plurality of sample MS images, also referred to as MS1 images, each sample image representing a proteome fulfilling a known first condition.

In the methods of the present invention, the neural network is preferably a convolutional neural network (CNN), a residual neural network (ResNet), a long short-term memory (LSTM), or a recurrent neural network (RNN), most preferred a CNN or a ResNet. Such neural networks are described e.g. in Loy Neural Network Projects with Python (Packt Publishing, 2019).

"First training" of the NN means that generic MS1 images representing a proteome fulfilling a known first binary (or multiclass) condition are used to teach the NN how to recognize and discriminate distinctive signals in MS1 maps. Preferably, said first condition of each sample image in the first plurality of sample MS1 images is a binary condition, where one of the conditions has features that are absent in the other condition or has features that are more/less intense than the corresponding features in the other condition. Distinguishing sample images within the first plurality of sample images, and said distinguishing features learned in the first training of the neural network, NN, facilitate subsequent second training of the NN and distinguishing conditions within the second plurality of sample images.

The term "Binary condition" as used herein means that two MS1 images comprising distinctive signals in each of the MS1 map, i.e. two classes of MS1 maps, are selected to teach the NN how to distinguish between the two sample images. In particular, the distinctive signals in each of the two MS1 maps refer to the presence/absence or differential abundance of certain molecules/biomarkers in said proteomes. Alternatively, it is envisaged that the first condition in the first plurality of sample MS1 images used to teach the NN how to discriminate between signals in said MS1 maps is a multiclass condition. "Multiclass condition" as used herein means that more than two classes, such as three, four or even more classes of MS1 maps comprising distinct features, e.g. one MS1 map from condition A, one MS1 map from condition B and one MS1 map from condition C, each comprising distinctive signals in the MS1 map, are used for the first training to teach the NN how to distinguish between said sample images.

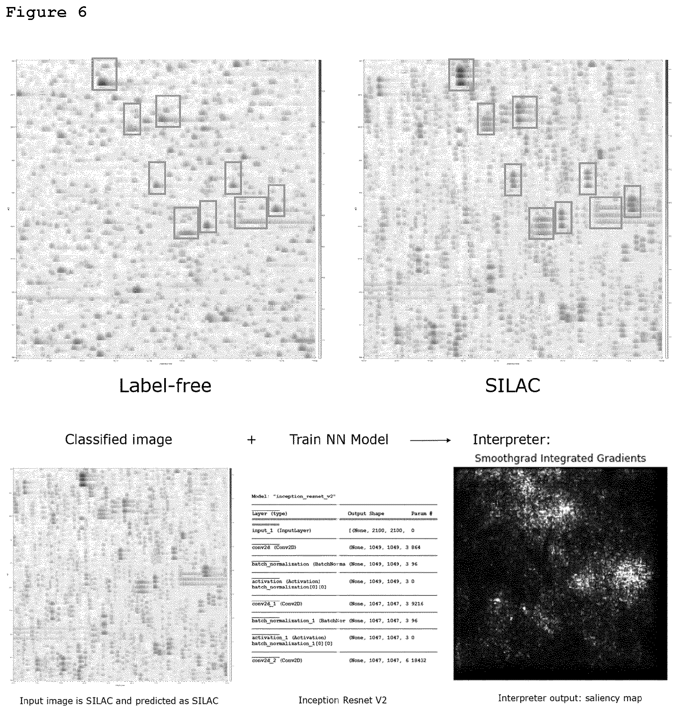

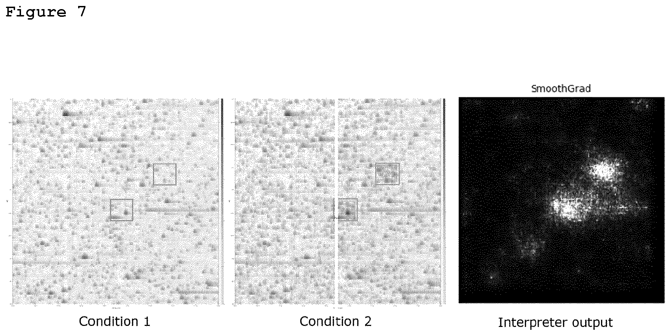

Hence, the first training of the NN can be based on a raw MS database of entire or partial proteomes of clinical or not clinical condition, proprietary or not. For example, SILAC versus Label-free, Cytoplasmic versus Nuclear proteome, or from Tissue A versus Tissue B proteome. Because the mentioned categories are quite generic, finding enough data (N>=1000) is not a limitation. Any of these data sets can be used as an input to the neural network during its first training. Said first training step allows the validation of the NN model because in any of the above mentioned data sets the distinctive peptide features between the classes is known in advance. If the Interpreter (e.g. the Grad-CAM defined elsewhere herein) pinpoints those peptide features, this is irrefutable proof that the NN model (i.e., the image classifier) learns correctly to distinguish classes based on their distinctive molecular features **(****Figure** 6 and **Figure** 7).

In the context of the methods of the present invention, the neural network has preferably one or more convolutional layers. Preferably, in the methods of the present invention, the neural network has one or more convolutional layers and at least one fully connected layer. The neural network can consist, for example, of 780 convolution layers, i.e. the model has 780 layers with several dozens of channels/kernels per layer as described herein in the Example section.

The channels can be two-dimensional or three-dimensional, depending on whether or not time series MS1 input data is used.

Preferably, in the methods of the present invention, each respective convolution layer has 128, 256, 512 or more kernels per layer with a shape of 200 by 4 per kernel.

A preferred neural network architecture consists of 780 layers.

The method of predicting a condition of health of a patient to be examined from MS specimen image of the present invention further comprises the step of a subsequent second training of the NN, via the input layer, with a second plurality of sample MS1 images from a plurality of patients, each sample image representing a proteome fulfilling a known second binary condition of health of a respective patient of the plurality of conditions, wherein the first training step of the NN facilitates the subsequent second training of the NN.

"Second training" of the NN means that said training is performed after the "first training" described elsewhere herein. In particular, MS1 images representing a proteome from a plurality of patients that fulfill a certain condition of health are used to teach the NN how to discriminate between signals in said MS1 maps from various patients. "Binary condition" means in this respect that two classes of MS1 maps comprising distinct features, e.g. one MS1 map from a healthy and one MS1 map from a diseased patient, each comprising distinctive signals in the MS1 map, are used for the second training to teach the NN how to distinguish between the two sample images. In this respect, it is highly desired to find distinctive features (presence, absence or differentialy abundant) in the second plurality of sample MS1 images as potential markers of two different conditions of health of a subject, such as health and disease. The "second binary condition of health" can be any condition of interest, i.e. can be selected from various known conditions of health. In this respect it is envisaged that various binary conditions of MS1 images representing the difference between two groups of patients are used. In this respect the NN is trained for a broad application, i.e. for predicting more than one condition of health.

Alternatively, in the context of the methods for predicting a condition of health of a patient to be examined of the present invention, the second training step may comprise multiclass conditions as described elsewhere herein. Hence, when in the first training step the NN is trained with a binary sample of images, the second training step can be done with a binary sample of images or a multiclass sample of images. Likewise, when in the first training step the NN is tranied with a multiclass sample of images, the second training step can be done with a binary sample of images or a multiclass sample of images. Multiclass conditions are e.g. tissue A versus tissue B versus tissue C; or disease stage 1 versus disease stage 2 versus disease stage 3, each comprising distinctive signals in the MS1 map that are utilized for the second training step. The "second multiclass condition" can be any condition of interest, i.e. can be selected from various known conditions of health that allows to differentiate between multiclass conditions. Also herein the NN is trained for a broad application, i.e. for predicting several conditions of health.

According to the method of the present invention, the first training step facilitates the second training of the NN. "Facilitates" means that the first training makes the second training easier and more precise, since the NN was already primed in the first training step how to differentiate between peptide features, i.e. distinct signals in MS1 maps. The second training step profits from said first training step, since the NN already knows how to find distinctive features (presence, absence or differentially abundant) in the MS1 images as potential markers of a condition of health (see **Figure 6** and **Figure 7****).** Hence, the second training step then allows for the actual clinical classification.

The requirement of the methods of the present invention that the first training of the NN facilitates the subsequent second training of the NN, is based on the following considerations.

Priming the NN in a first training step with a first plurality of non-scarce MS1 images has the aim of optimizing the node weights for the inner layers of the NN. Because this first sample of images is not scarce (N>1000 per class), properly training the NN is not a problem. After this first training, the very last layer (i.e., the output layer) of the trained NN model is removed and replaced by an untrained output layer that will be trained with images of the second training round. Because the images from the first training are essentially of the same kind as those from the second training (i.e., both are proteomic images), weights learnt during the first training can be transfered to the second training. However, the classification task is different: in the first training, the NN learns to differentiate e.g. SILAC from Label-free images, whereas in the second training the NN learns to differentiate between e.g., Breast Cancer and Healthy conditions. Because the two classification tasks are different, the output layer from the first training is left aside and replaced by a new untrained output layer. As a result, only the outermost layer needs to be trained now and, therefore, less training data is required (N<=500 per class).

After the second training step of the NN, the method of the present invention provides, via an output layer of the NN, a prediction of a condition of health of the patient to be examined. Subsequently, the method of the invention finally employs an Saliency Map tool, also referred as the Interpreter, to pinpoint the specific features in the image that distinguish the binary conditions. Hence, based on the MS image representing a proteome, the condition of health of said patient can be defined as well as the molecular biomarkers of the condition.

In preferred embodiments of the method of the present invention it is particularly envisaged that, the first condition of each sample image in the first plurality of sample MS1 images is a binary condition designating SILAC-labelled versus Label-free MS1 images, respectively, and wherein the second condition of each sample MS1 image in the second plurality of sample images from the plurality of patients, represents a binary condition of diseased or healthy, respectively, of said patient to be examined. As depicted in **Figure 3****,** SILAC-label MS data contain distinctive features that are not present in label-free MS1 images, i.e. the NN shall learn in such a dataset to distinguish presence/absence of peptide signals **(****Figure 6****),** thereby identifying unique peptide features in the two types of MS images.

"SILAC-labelled" MS images are based on stable isotope labeling with amino acids in cell culture (SILAC). Contrary thereto, "label-free" MS aims to determine the relative amount of proteins, thereby not using a stable isotope containing compound to chemically bind to and thus label the protein. Hence, usage of SILAC-labelled and label-free MS1 images as binary condition for the first training steps allows the NN to generally differentiate between peptide features, i.e. distinct signals, in the two MS1 maps which will then facilitate the second training step, i.e. the differentiation between MS1 maps from diseased or healthy patients in a second plurality of samples. Said second training step finally allows to classify the MS1 map from the patient to be examined into one of the two group, i.e. to predict whether said patient is healthy or diseased. Other binary conditions useful for the first training are e.g. Tissue

A versus Tissue B; or Cell-line A proteome versus Cell-line B; or a Cytoplasmic proteome versus and Nuclear proteome.

However, in addition to cases which provide presence and absence of certain features, also differentially intense signals can be used as binary (or multiclass) condition. In this case the signal is present in both/all classes/conditions, but is more intense/abundant in one condition relative to the other, and the interpreter is equally able to highlight those differentially expressed regions in simulated images **(****Figure 7****).** In this respect differentially abundant features in MS maps can be used as potential markers of a binary (or multiclass) condition/disease of a subject.

The present invention also refers to a computer program product adapted to carry out the method as described herein above.

The computer program product may use hardware, firmware, software or a combination thereof to perform the various steps and operations described herein.

A computing system suitable for performing the activities described herein may include a server. Such a server may include a central processor (CPU) coupled to a random-access memory (RAM) and to a read-only memory (ROM). ROM may also be other types of storage media to store programs, such as programmable ROM, erasable PROM, etc. Processor may communicate with other internal and external components through input/output (I/O) circuitry and bussing to provide control signals and the like. Processor carries out a variety of functions as are known in the art, as dictated by software and/or firmware instructions.

Server may also include one or more data storage devices, including hard drives, CD-ROM drives and other hardware capable of reading and/or storing information, such as DVD, etc. In one embodiment, software for carrying out the herein described steps may be stored and distributed on a CD-ROM or DVD, a USB storage device or other form of media capable of portably storing information. These storage media may be inserted into, and read by, devices such as CD-ROM drive, disk drive, etc. Server may be coupled to a display, which may be any type of known display or presentation screen, such as LCD, plasma display, cathode ray tube (CRT), etc. A user input interface is provided, including one or more user interface mechanisms such as a mouse, keyboard, microphone, touchpad, touch screen, voice-recognition system, etc.

Server may be coupled to other devices, such as sources, detectors, etc. The server may be part of a larger network configuration as in a global area network, such as the Internet, which allows ultimate connection to various landline and/or mobile computing devices.

### Description of the Figures:

**Figure 1****: 2D representation of MS1 map**
   MS1 map in 2D representation (left side). The Y axis indicates the mass and the X axis the elution time of a molecule, whereas a value of a pixel reflects the abundance of the molecule. A group of pixels represent a molecule and the white/yellow/red colour the intensity of its signal (i.e. its abundance in the sample). The zoom-in on the right side is a 3D representation of a region in the map and depicts each molecule as a peak.
**Figure** 2: **Portion of a MS1 map with 4 signals pinpointed.** The relative elution time of the signals from each other is a characteristic that current biomarker discovery methods do not exploit. Additionally, the shape of each signal is also different from peptide to peptide; some signals being more symmetric (1 & 2) whereas others have longer tails (3 & 4).
**Figure 3****: Two MS1 map portions from Label-free (left) and SILAC data (right).** Comparing the images, shows that SILAC data contains distinctive features that its counterpart does not have. A CNN/ResNet model shall learn, in such a dataset, to distinguish presence/absence of peptide signals (or also called unique peptide features). Common peptide features are indicated with the letter "L", unique SILAC features with the letters "M" and "H".
**Figure 4****: Deep Learning modelling versus machine learning approach.** The benchmark of our Deep Learning (DL) modelling approach versus the classical machine learning approach that uses protein abundance matrices results in better prediction accuracies and sensitivities for the DL model.
**Figure** 5: **Overview workflow of the AI platform for the binary classification of MS1 maps and the recognition of discriminative features.** Data acquisition starts by preparation of the biological/clinical sample for mass spectrometry quantification. The mass spectrometer outputs a data file, called RAW data, per patient (or sample) which is converted into an image (step 1). This data file is the starting point of the platform used according to the method of the present invention. A neural network model is first trained in generic data (step 2) and then trained in data of a condition of health (step 3). Finally, the trained model and the classified images are passed to an Interpreter (i.e. a Saliency map method; e.g., Grad-CAM) to pinpoint the distinctive features and regions in the images that distinguish the two conditions of health from one another.
**Figure 6****: First training on a binary dataset of SILAC vs Label-free images that leads the NN to learn the presence and absence of distinctive features.** In the top panel, the MS1 maps of a Label-free and a SILAC conditions are shown. Some of the distinctive regions between the two conditions are pointed out with rectangles. In the bottom panel, a predicted SILAC image plus the model is fed to the Model Interpreter (in this case a SmoothGrad), which outputs a Saliency map that proves that the NN model has learned to differentiate some distinctive regions (highlighted in white) in the SILAC map relative to the Label-free.
**Figure** 7: **First training on a binary dataset that leads the NN to learn differentially abundant features.** In this example, the NN model was trained with original Label-free images and augmented Label-free images. In the latter class, some regions within the MS1 map were intensity-enhanced in order to simulate differentially abundant molecular features. After training, the Model Interpreter shows that indeed the NN model learns to distinguish features that are present in both classes, but that are differentially abundant.

### Examples

### Example 1

Given are two starting binary data sets of MS1 images, where the first data set comprises certain markers that are present in one condition and absent in the other condition **(****Figure 6****,** top panel MS1 maps), and the second data set where certain markers are present in both conditions, but more abundantly in one condition relative to the other condition **(****Figure 7****,** first two MS1 maps). These two data sets describe (or emulate) a similar scenario as the one observed in a clinical application of biomarker discovery, because in such a scenerario some biomarkers of the disease will be present/absent only in the disease or will be present in both, disease and healthy groups, but in different abundance (for some markers their abundance will be higher in the diseased group, for some other markers it will be lower in the diseased group).

These two data sets have been used to train 3 independent neural networks: "Inception ResNet v2" (780 layers; 54336736 parameters); ResNet50 (48 convolution layers; Y trainable parameters); or EfficientNet B4 (459 layers; 18830011 trainable parameters). For the first training, data set one was used, 945 images from each class was fed to the NN. The optimizers used during training were either Stochastic Gradient Descent, SGD, or Root Mean Squared Propagation, RMSprop, with a learning rate of 0.001-0.0001. For monitoring training in each epoch, the mini-batch method was used with batch sizes of 10, 16, 16 (respectively to the 3 NN models named above). After 20, 10, 40 epochs (for the 3 NN's respectively named above; and equivalent to <=1 day of training), the achieved accuracy level for any of the models was 97-98% in the validation set and 95-97% in the test set.

Once the NN was trained in the first data set, a SILAC image (so far unseen by the model) was given to the NN for prediction. The model predicted the image correctly as SILAC. However, as expected and known, the NN is unable to explain on which basis this decision was taken. Thus, Model Interpreters from a set of Saliency Methods were selected. The best performing interpreters were SmoothGrad (D. Smilkov et al., 2017 arXiv:1706.03825). To increase the accuracy and resolution of SmoothGrad in telling the distinctive regions within the MS1 map, SmoothGrad was improved using NoiseGrad (K. Bykov et al., 2021 arXiv:2106.10185) at the same time, while to increase the time-efficiency of the Model Interpreter the FastCAM approach (T.N. Mundhenk et al., 2019, arXiv:1911.11293) was used. The Model Interpreter receives as input the trained NN model plus the classified image and then outputs the regions within the image that were more important for the NN model to learn the discrimination of the two classes in the dataset. In this way, the Model Interpreter, explains the decision of the NN model. In the context of this example, **Figure 6** (bottom panel) shows that the image was classified as SILAC by the NN due to molecular features within the white-highlighted regions in the image.

In a next training step, the last layer of the trained NN model was removed and replaced by a new, untrained layer while the rest of the layers remained untouched. This modified NN model was then trained again using this time the second data set of images (i.e., the set where one of the conditions has low abundant features and the other condition has highly abundant features; **Figure 7****;** 108 images in each of the two classes). After training, the new NN model was able to distinguish the two conditions with high accuracy (>98.5%)in both the validation and test sets. When the new NN model and classified image where fed to the Model Interpreter, this was able to highlight the regions where indeed the two conditions differ from each other. In other words, the NN model is able to learn to distinguish the conditions based on their distinctive molecular features and the Model Interpreter is able to pinpoint, to the human user, those regions within the image that contain the discriminative molecular features **(****Figure 7****).**

### Example 2

In practice, a laboratory in possession of a license for using the web/computer/mobile software application of the method described herein uses the software application to convert the raw data that comes from the LC-MS to an MS1 image (i.e. MS1 map proteomic profile of the patient). Subsequently, the software application utilizes the trained NN described somewhere else herein to predict the condition of the patient. A pre-requisite, of course, is that the NN model has been trained to recognize proteome images of a certain disease, e.g. colorectal cancer MS1 proteome images. Subsequently the Interpreter pinpoints the biomarkers which lead to this prediction. The software application provides back to the laboratory technician the MS1 image with the distinctive regions highlighted plus a list of the corresponding masses and elution times of those regions. The laboratory technician confirms, using the raw file, that indeed the identity of the biomarkers matches with the ones learned during training of the DL platform. The laboratory, finally, provides the medical doctor with the MS1 image annotated with the predicted condition of the patient as well as the ID-ed and quantity levels of the biomarkers observed in the patient sample.

### Example of a ResNet model trained for SILAC versu Label-free classification as described in Example 1

**Model: "inception_resnet_v2"**

| L̅a̅y̅e̅r̅ (̅t̅y̅pe) | Output Shape | Param # | Connected to |
|---|---|---|---|
| input_1 (InputLayer) | [(None, 2100, 2100, | 0 | |
| c̅o̅n̅v̅2̅d̅ (̅C̅onv2D) | (None, 1049, 1049, 3 | 864 | input_1[0][0] |
| b̅a̅t̅c̅h̅_̅n̅o̅r̅malization (BatchNorma | (None, 1049, 1049, 3 | 96 | conv2d[0][0] |
| a̅c̅t̅i̅v̅a̅t̅i̅o̅n̅ (Activation) | (None, 1049, 1049, 3 | 0 | |
| b̅a̅t̅c̅h̅_̅n̅o̅r̅malization[0][0] | | | |
| c̅o̅n̅v̅2̅d̅_̅1̅ (Conv2D) | (None, 1047, 1047, 3 | 9216 | activation[0][0] |
| b̅a̅t̅c̅h̅_̅n̅o̅r̅m̅alization_1 (BatchNor | (None, 1047, 1047, 3 | 96 | conv2d_1[0][0] |
| a̅c̅t̅i̅v̅a̅t̅i̅o̅n_1 (Activation) | (None, 1047, 1047, 3 | 0 | |
| batch_normalization_1[0][0] | | | |
| c̅o̅n̅v̅2̅d̅_̅2̅ (Conv2D) | (None, 1047, 1047, 6 | 18432 | activation_1[0][0] |
| b̅a̅t̅c̅h̅_̅n̅o̅r̅malization_2 (BatchNor | (None, 1047, 1047, 6 | 192 | conv2d_2[0][0] |
| a̅c̅t̅i̅v̅a̅t̅i̅o̅n_2 (Activation) | (None, 1047, 1047, 6 | 0 | |
| batch_normalization_2[0][0] | | | |
| m̅a̅x̅_̅p̅o̅o̅l̅i̅ng2d (MaxPooling2D) | (None, 523, 523, 64) | 0 | activation_2[0][0] |
| c̅o̅n̅v̅2̅d̅_̅3̅ (Conv2D) | (None, 523, 523, 80) | 5120 | max_pooling2d[0][0] |
| b̅a̅t̅c̅h̅_̅n̅o̅r̅malization_3 (BatchNor | (None, 523, 523, 80) | 240 | conv2d_3[0][0] |
| a̅c̅t̅i̅v̅a̅t̅i̅o̅n_3 (Activation) | (None, 523, 523, 80) | 0 | |
| batch_normalization_3[0][0] | | | |
| c̅o̅n̅v̅2̅d̅_̅4̅ (Conv2D) | (None, 521, 521, 192 | 138240 | activation_3[0][0] |
| b̅a̅t̅c̅h̅_̅n̅o̅r̅malization_4 (BatchNor | (None, 521, 521, 192 | 576 | conv2d_4[0][0] |
| a̅c̅t̅i̅v̅a̅t̅i̅o̅n_4 (Activation) | (None, 521, 521, 192 | 0 | |
| batch_normalization_4[0][0] | | | |
| m̅a̅x̅ p̅o̅o̅l̅i̅ng2d_1 (MaxPooling2D) | (None, 260, 260, 192 | 0 | activation_4[0][0] |
| c̅o̅n̅v̅2̅d̅_̅8̅ (Conv2D) | (None, 260, 260, 64) | 12288 | max_pooling2d_1[0][0] |
| b̅a̅t̅c̅h̅_̅n̅o̅r̅malization_8 (BatchNor | (None, 260, 260, 64) | 192 | conv2d_8 [0] [0] |
| a̅c̅t̅i̅v̅a̅t̅i̅o̅n_8 (Activation) | (None, 260, 260, 64) | 0 | |
| batch_normalization_8[0][0] | | | |
| c̅o̅n̅v̅2̅d̅_̅6̅ (Conv2D) | (None, 260, 260, 48) | 9216 | max_pooling2d_1[0] [0] |
| c̅o̅n̅v̅2̅d̅_̅9̅ (Conv2D) | (None, 260, 260, 96) | 55296 | activation_[0][0] |
| b̅a̅t̅c̅h̅_̅n̅o̅r̅malization_6 (BatchNor | (None, 260, 260, 48) | 144 | conv2d_6[0][0] |
| b̅a̅t̅c̅h̅_̅n̅o̅r̅malization_9 (BatchNor | (None, 260, 260, 96) | 288 | conv2d_9[0][0] |
| a̅c̅t̅i̅v̅a̅t̅i̅o̅n_6 (Activation) | (None, 260, 260, 48) | 0 | |
| batch_normalization_6[0][0] | | | |
| a̅c̅t̅i̅v̅a̅t̅i̅o̅n_9 (Activation) | (None, 260, 260, 96) | 0 | |
| batch_normalization_9[0][0] | | | |
| a̅v̅e̅r̅a̅g̅e̅_̅p̅o̅oling2d (AveragePooli | (None, 260, 260, 192 | 0 | max_pooling2d_1[0][0] |
| c̅o̅n̅v̅2̅d̅_̅5̅ (Conv2D) | (None, 260, 260, 96) | 18432 | max_pooling2d_1[0][0] |
| c̅o̅n̅v̅2̅d̅_̅7̅ (Conv2D) | (None, 260, 260, 64) | 76800 | activation_6[0][0] |
| c̅o̅n̅v̅2̅d̅_̅1̅0̅ (Conv2D) | (None, 260, 260, 96) | 82944 | activation_9[0] [0] |
| c̅o̅n̅v̅2̅d̅_̅1̅1̅ (Conv2D) | (None, 260, 260, 64) | 12288 | average_pooling2d[0][0] |
| b̅a̅t̅c̅h̅_̅n̅o̅r̅malization_5 (BatchNor | (None, 260, 260, 96) | 288 | conv2d_5[0][0] |
| b̅a̅t̅c̅h̅_̅n̅o̅r̅malization_7 (BatchNor | (None, 260, 260, 64) | 192 | conv2d_7[0][0] |
| b̅a̅t̅c̅h̅_̅n̅o̅r̅malization_10 (BatchNo | (None, 260, 260, 96) | 288 | conv2d_10[0][0] |
| b̅a̅t̅c̅h̅_̅n̅o̅r̅malization_11 (BatchNo | (None, 260, 260, 64) | 192 | conv2d_11[0][0] |
| a̅c̅t̅i̅v̅a̅t̅i̅o̅n_5 (Activation) | (None, 260, 260, 96) | 0 | |
| batch_normalization_5[0][0] | | | |
| a̅c̅t̅i̅v̅a̅t̅i̅o̅n_7 (Activation) | (None, 260, 260, 64) | 0 | |
| batch_normalization_7[0][0] | | | |
| a̅c̅t̅i̅v̅a̅t̅i̅o̅n 10 (Activation) | (None, 260, 260, 96) | 0 | |
| batch_normalization_10[0][0] | | | |
| a̅c̅t̅i̅v̅a̅t̅i̅o̅n_11 (Activation) | (None, 260, 260, 64) | 0 | |
| batch_normalization_11[0][0] | | | |
| m̅i̅x̅e̅d̅_̅5̅b̅ (Concatenate) | (None, 260, 260, 320 | 0 | activation_5[0] [0] |
| | | | activation_7[0] [0] |
| | | | activation_10[0] [0] |
| | | | activation_11[0] [0] |
| c̅o̅n̅v̅2̅d̅_̅1̅5̅ (Conv2D) | (None, 260, 260, 32) | 10240 | mixed_5b[0] [0] |
| b̅a̅t̅c̅h̅_̅n̅o̅r̅malization_15 (BatchNo | (None, 260, 260, 32) | 96 | conv2d_15[0] [0] |
| a̅c̅t̅i̅v̅a̅t̅i̅o̅n_15 (Activation) | (None, 260, 260, 32) | 0 | |
| batch_normalization_15[0][0] | | | |
| c̅o̅n̅v̅2̅d̅_̅1̅3̅ (Conv2D) | (None, 260, 260, 32) | 10240 | mixed_5b[0] [0] |
| c̅o̅n̅v̅2̅d̅_̅1̅6̅ (Conv2D) | (None, 260, 260, 48) | 13824 | activation_15[0] [0] |
| b̅a̅t̅c̅h̅_̅n̅o̅r̅malization_13 (BatchNo | (None, 260, 260, 32) | 96 | conv2d_13[0] [0] |
| b̅a̅t̅c̅h̅_̅n̅o̅r̅malization_16 (BatchNo | (None, 260, 260, 48) | 144 | conv2d_16[0] [0] |
| a̅c̅t̅i̅v̅a̅t̅i̅o̅n_13 (Activation) | (None, 260, 260, 32) | 0 | |
| batch_norma̅lization_13[0][0] | | | |
| a̅c̅t̅i̅v̅a̅t̅i̅o̅n_16 (Activation) | (None, 260, 260, 48) | 0 | |
| batch_normalization_16[0][0] | | | |
| c̅o̅n̅v̅2̅d̅_̅1̅2̅ (Conv2D) | (None, 260, 260, 32) | 10240 | mixed_5b[0][0] |
| c̅o̅n̅v̅2̅d̅_̅1̅4̅ (Conv2D) | (None, 260, 260, 32) | 9216 | activation_13[0] [0] |
| c̅o̅n̅v̅2̅d̅_̅1̅7̅ (Conv2D) | (None, 260, 260, 64) | 27648 | activation_16[0] [0] |
| b̅a̅t̅c̅h̅_̅n̅o̅r̅malization_12 (BatchNo | (None, 260, 260, 32) | 96 | conv2d_12[0] [0] |
| b̅a̅t̅c̅h̅_̅n̅o̅r̅malization_14 (BatchNo | (None, 260, 260, 32) | 96 | conv2d_14[0] [0] |
| b̅a̅t̅c̅h̅_̅n̅o̅r̅malization_17 (BatchNo | (None, 260, 260, 64) | 192 | conv2d_17[0] [0] |
| a̅c̅t̅i̅v̅a̅t̅i̅o̅n_12 (Activation) | (None, 260, 260, 32) | 0 | |
| batch_norma̅lization_12[0][0] | | | |
| a̅c̅t̅i̅v̅a̅t̅i̅o̅n_14 (Activation) | (None, 260, 260, 32) | 0 | |
| batch_normalization_14[0] [0] | | | |
| a̅c̅t̅i̅v̅a̅t̅i̅o̅n_17 (Activation) | (None, 260, 260, 64) | 0 | |
| batch_normalization_17[0][0] | | | |
| b̅l̅o̅c̅k̅3̅5̅_̅1̅_̅mixed (Concatenate) | (None, 260, 260, 128 | 0 | activation_12[0] [0] |
| | | | activation_14[0] [0] |
| | | | activation_17[0] [0] |
| b̅l̅o̅c̅k̅3̅5̅_̅1̅_conv (Conv2D) | (None, 260, 260, 320 | 41280 | block35_1_mixed[0][0] |
| b̅l̅o̅c̅k̅3̅5̅_̅1̅ (Lambda) | (None, 260, 260, 320 | 0 | mixed_5b[0][0] block35_1_conv[0][0] |
| b̅l̅o̅c̅k̅3̅5̅_̅1̅_ac (Activation) | (None, 260, 260, 320 | 0 | block35_1[0] [0] |
| c̅o̅n̅v̅2̅d̅_̅2̅1̅ (Conv2D) | (None, 260, 260, 32) | 10240 | block35_1_ac[0][0] |
| b̅a̅t̅c̅h̅_̅n̅o̅r̅malization_21 (BatchNo | (None, 260, 260, 32) | 96 | conv2d_21[0][0] |
| a̅c̅t̅i̅v̅a̅t̅i̅o̅n_21 (Activation) | (None, 260, 260, 32) | 0 | |
| batch_normalization_21[0] [0] | | | |
| c̅o̅n̅v̅2̅d̅_̅1̅9̅ (Conv2D) | (None, 260, 260, 32) | 10240 | block35_1_ac[0][0] |
| c̅o̅n̅v̅2̅d̅_̅2̅2̅ (Conv2D) | (None, 260, 260, 48) | 13824 | activation_21[0][0] |
| b̅a̅t̅c̅h̅_̅n̅o̅r̅malization_19 (BatchNo | (None, 260, 260, 32) | 96 | conv2d_19[0] [0] |
| b̅a̅t̅c̅h̅_̅n̅o̅r̅malization_22 (BatchNo | (None, 260, 260, 48) | 144 | conv2d_22[0][0] |
| a̅c̅t̅i̅v̅a̅t̅i̅o̅n_19 (Activation) | (None, 260, 260, 32) | 0 | |
| batch_normalization_19 [0] [0] | | | |
| a̅c̅t̅i̅v̅a̅t̅i̅o̅n_22 (Activation) | (None, 260, 260, 48) | 0 | |
| batch_normalization_22[0] [0] | | | |
| c̅o̅n̅v̅2̅d̅_̅1̅8̅ (Conv2D) | (None, 260, 260, 32) | 10240 | block35_1_ac[0][0] |
| c̅o̅n̅v̅2̅d̅_̅2̅0̅ (Conv2D) | (None, 260, 260, 32) | 9216 | activation_19[0][0] |
| c̅o̅n̅v̅2̅d̅_̅2̅3̅ (Conv2D) | (None, 260, 260, 64) | 27648 | activation_22[0][0] |
| b̅a̅t̅c̅h̅_̅n̅o̅r̅malization_18 (BatchNo | (None, 260, 260, 32) | 96 | conv2d_18[0][0] |
| b̅a̅t̅c̅h̅_̅n̅o̅r̅malization_20 (BatchNo | (None, 260, 260, 32) | 96 | conv2d_20[0] [0] |
| b̅a̅t̅c̅h̅_̅n̅o̅r̅m̅alization_23 (BatchNo | (None, 260, 260, 64) | 192 | conv2d_23[0] [0] |
| a̅c̅t̅i̅v̅a̅t̅i̅o̅n_18 (Activation) | (None, 260, 260, 32) | 0 | |
| batch_normalization_18[0] [0] | | | |
| a̅c̅t̅i̅v̅a̅t̅i̅o̅n_20 (Activation) | (None, 260, 260, 32) | 0 | |
| batch_normalization_20[0] [0] | | | |
| a̅c̅t̅i̅v̅a̅t̅i̅o̅n_23 (Activation) | (None, 260, 260, 64) | 0 | |
| batch_normalization_23[0] [0] | | | |
| b̅l̅o̅c̅k̅3̅5̅_̅2̅_mixed (Concatenate) | (None, 260, 260, 128 | 0 | activation_18 [0][0] |
| | | | activation_20[0][0] |
| | | | activation_23[0][0] |
| b̅l̅o̅c̅k̅3̅5̅_̅2̅_conv (Conv2D) | (None, 260, 260, 320 | 41280 | block35_2_mixed[0] [0] |
| b̅l̅o̅c̅k̅3̅5̅_̅2̅ (Lambda) | (None, 260, 260, 320 | 0 | block35_1_ac[0] [0] block35_2_conv[0] [0] |
| b̅l̅o̅c̅k̅3̅5̅_̅2̅_ac (Activation) | (None, 260, 260, 320 | 0 | block35_2[0] [0] |
| c̅o̅n̅v̅2̅d̅_̅2̅7̅ (Conv2D) | (None, 260, 260, 32) | 10240 | block35_2_ac[0][0] |
| b̅a̅t̅c̅h̅_̅n̅o̅r̅malization_27 (BatchNo | (None, 260, 260, 32) | 96 | conv2d_27[0][0] |
| a̅c̅t̅i̅v̅a̅t̅i̅o̅n_27 (Activation) | (None, 260, 260, 32) | 0 | |
| batch_normalization_27[0][0] | | | |
| c̅o̅n̅v̅2̅d̅_̅2̅5̅ (Conv2D) | (None, 260, 260, 32) | 10240 | block35_2_ac[0] [0] |
| c̅o̅n̅v̅2̅d̅_̅2̅8̅ (Conv2D) | (None, 260, 260, 48) | 13824 | activation_27[0][0] |
| b̅a̅t̅c̅h̅_̅n̅o̅r̅malization_25 (BatchNo | (None, 260, 260, 32) | 96 | conv2d_25[0] [0] |
| batch_normalization_28 (BatchNo | (None, 260, 260, 48) | 144 | conv2d_28[0][0] |
| a̅c̅t̅i̅v̅a̅t̅i̅o̅n_25 (Activation) | (None, 260, 260, 32) | 0 | |
| batch_normalization_25[0] [0] | | | |
| a̅c̅t̅i̅v̅a̅t̅i̅o̅n_28 (Activation) | (None, 260, 260, 48) | 0 | |
| batch_normalization_28[0][0] | | | |
| c̅o̅n̅v̅2̅d̅_̅2̅4̅ (Conv2D) | (None, 260, 260, 32) | 10240 | block35_2_ac[0] [0] |
| c̅o̅n̅v̅2̅d̅_̅2̅6̅ (Conv2D) | (None, 260, 260, 32) | 9216 | activation_25[0][0] |
| c̅o̅n̅v̅2̅d̅_̅2̅9̅ (Conv2D) | (None, 260, 260, 64) | 27648 | activation_28[0][0] |
| b̅a̅t̅c̅h̅_̅n̅o̅r̅malization_24 (BatchNo | (None, 260, 260, 32) | 96 | conv2d_24[0] [0] |
| b̅a̅t̅c̅h̅_̅n̅o̅r̅malization_26 (BatchNo | (None, 260, 260, 32) | 96 | conv2d_26[0] [0] |
| b̅a̅t̅c̅h̅_̅n̅o̅r̅malization_29 (BatchNo | (None, 260, 260, 64) | 192 | conv2d_29[0] [0] |
| a̅c̅t̅i̅v̅a̅t̅i̅o̅n_24 (Activation) | (None, 260, 260, 32) | 0 | |
| batch_normalization_24[0][0] | | | |
| a̅c̅t̅i̅v̅a̅t̅i̅o̅n_26 (Activation) | (None, 260, 260, 32) | 0 | |
| batch_normalization_26[0][0] | | | |
| a̅c̅t̅i̅v̅a̅t̅i̅o̅n_29 (Activation) | (None, 260, 260, 64) | 0 | |
| batch_normalization_29[0][0] | | | |
| b̅l̅o̅c̅k̅3̅5̅_̅3̅_mixed (Concatenate) | (None, 260, 260, 128 | 0 | activation_24[0] [0] |
| | | | activation_26[0] [0] |
| | | | activation_29[0] [0] |
| b̅l̅o̅c̅k̅3̅5̅_̅3̅_conv (Conv2D) | (None, 260, 260, 320 | 41280 | block35_3_mixed[0] [0] |
| b̅l̅o̅c̅k̅3̅5̅_̅3̅ (Lambda) | (None, 260, 260, 320 | 0 | block35_2_ac[0] [0] |
| | | | block35_3_conv[0] [0] |
| b̅l̅o̅c̅k̅3̅5̅_̅3̅_ac (Activation) | (None, 260, 260, 320 | 0 | block35_3[0] [0] |
| c̅o̅n̅v̅2̅d̅_̅3̅3̅ (Conv2D) | (None, 260, 260, 32) | 10240 | block35_3_ac[0] [0] |
| b̅a̅t̅c̅h̅_̅n̅o̅r̅malization_33 (BatchNo | (None, 260, 260, 32) | 96 | conv2d_33[0] [0] |
| a̅c̅t̅i̅v̅a̅t̅i̅o̅n_33 (Activation) | (None, 260, 260, 32) | 0 | |
| batch_normalization_33 [0] [0] | | | |
| c̅o̅n̅v̅2̅d̅_̅3̅1̅ (Conv2D) | (None, 260, 260, 32) | 10240 | block35_3_ac[0] [0] |
| c̅o̅n̅v̅2̅d̅_̅3̅4̅ (Conv2D) | (None, 260, 260, 48) | 13824 | activation_33 [0] [0] |
| b̅a̅t̅c̅h̅_̅n̅o̅r̅malization_31 (BatchNo | (None, 260, 260, 32) | 96 | conv2d_31[0] [0] |
| b̅a̅t̅c̅h̅_̅n̅o̅r̅m̅alization_34 (BatchNo | (None, 260, 260, 48) | 144 | conv2d_34[0] [0] |
| a̅c̅t̅i̅v̅a̅t̅i̅o̅n_31 (Activation) | (None, 260, 260, 32) | 0 | |
| batch_norma̅lization_31[0][0] | | | |
| a̅c̅t̅i̅v̅a̅t̅i̅o̅n_34 (Activation) | (None, 260, 260, 48) | 0 | |
| batch_normalization_34 [0] [0] | | | |
| c̅o̅n̅v̅2̅d̅_̅3̅0̅ (Conv2D) | (None, 260, 260, 32) | 10240 | block35_3_ac[0] [0] |
| c̅o̅n̅v̅2̅d̅_̅3̅2̅ (Conv2D) | (None, 260, 260, 32) | 9216 | activation_31[0] [0] |
| c̅o̅n̅v̅2̅d̅_̅3̅5̅ (Conv2D) | (None, 260, 260, 64) | 27648 | activation_34 [0] [0] |
| b̅a̅t̅c̅h̅_̅n̅o̅r̅malization_30 (BatchNo | (None, 260, 260, 32) | 96 | conv2d_30[0] [0] |
| b̅a̅t̅c̅h̅_̅n̅o̅r̅malization_32 (BatchNo | (None, 260, 260, 32) | 96 | conv2d_32[0] [0] |
| b̅a̅t̅c̅h̅_̅n̅o̅r̅malization_35 (BatchNo | (None, 260, 260, 64) | 192 | conv2d_35[0] [0] |
| a̅c̅t̅i̅v̅a̅t̅i̅o̅n_30 (Activation) | (None, 260, 260, 32) | 0 | |
| batch_normalization_30[0] [0] | | | |
| a̅c̅t̅i̅v̅a̅t̅i̅o̅n_32 (Activation) | (None, 260, 260, 32) | 0 | |
| batch_normalization_32[0] [0] | | | |
| a̅c̅t̅i̅v̅a̅t̅i̅o̅n_35 (Activation) | (None, 260, 260, 64) | 0 | |
| batch_normalization_35[0] [0] | | | |
| b̅l̅o̅c̅k̅3̅5̅_̅4̅_mixed (Concatenate) | (None, 260, 260, 128 | 0 | activation_30[0] [0] |
| | | | activation_32[0] [0] |
| | | | activation_35[0] [0] |
| b̅l̅o̅c̅k̅3̅5̅_̅4̅_conv (Conv2D) | (None, 260, 260, 320 | 41280 | block35_4_mixed[0] [0] |
| b̅l̅o̅c̅k̅3̅5̅_̅4̅ (Lambda) | (None, 260, 260, 320 | 0 | block35_3_ac[0] [0] |
| | | | block35_4_conv[0] [0] |
| b̅l̅o̅c̅k̅3̅5̅_̅4̅_ac (Activation) | (None, 260, 260, 320 | 0 | block35_4[0] [0] |
| c̅o̅n̅v̅2̅d̅_̅3̅9̅ (Conv2D) | (None, 260, 260, 32) | 10240 | block35_4_ac[0] [0] |
| b̅a̅t̅c̅h̅_̅n̅o̅r̅malization_39 (BatchNo | (None, 260, 260, 32) | 96 | conv2d_39[0] [0] |
| a̅c̅t̅i̅v̅a̅t̅i̅o̅n_39 (Activation) | (None, 260, 260, 32) | 0 | |
| batch_normalization_39[0] [0] | | | |
| c̅o̅n̅v̅2̅d̅_̅3̅7̅ (Conv2D) | (None, 260, 260, 32) | 10240 | block35_4_ac[0] [0] |
| c̅o̅n̅v̅2̅d̅_̅4̅0̅ (Conv2D) | (None, 260, 260, 48) | 13824 | activation_39 [0] [0] |
| b̅a̅t̅c̅h̅_̅n̅o̅r̅malization_37 (BatchNo | (None, 260, 260, 32) | 96 | conv2d_37[0] [0] |
| b̅a̅t̅c̅h̅_̅n̅o̅r̅malization_40 (BatchNo | (None, 260, 260, 48) | 144 | conv2d_40[0] [0] |
| a̅c̅t̅i̅v̅a̅t̅i̅o̅n_37 (Activation) | (None, 260, 260, 32) | 0 | |
| batch_normalization_37[0] [0] | | | |
| a̅c̅t̅i̅v̅a̅t̅i̅o̅n_40 (Activation) | (None, 260, 260, 48) | 0 | |
| batch_normalization_40[0] [0] | | | |
| c̅o̅n̅v̅2̅d̅_̅3̅6̅ (Conv2D) | (None, 260, 260, 32) | 10240 | block35_4_ac[0] [0] |
| c̅o̅n̅v̅2̅d̅_̅3̅8̅ (Conv2D) | (None, 260, 260, 32) | 9216 | activation_37 [0] [0] |
| c̅o̅n̅v̅2̅d̅_̅4̅1̅ (Conv2D) | (None, 260, 260, 64) | 27648 | activation_40 [0] [0] |
| b̅a̅t̅c̅h̅_̅n̅o̅r̅malization_36 (BatchNo | (None, 260, 260, 32) | 96 | conv2d_36[0] [0] |
| b̅a̅t̅c̅h̅_̅n̅o̅r̅malization_38 (BatchNo | (None, 260, 260, 32) | 96 | conv2d_38[0] [0] |
| b̅a̅t̅c̅h̅_̅n̅o̅r̅malization_41 (BatchNo | (None, 260, 260, 64) | 192 | conv2d_41[0] [0] |
| a̅c̅t̅i̅v̅a̅t̅i̅o̅n_36 (Activation) | (None, 260, 260, 32) | 0 | |
| batch_normalization_36[0] [0] | | | |
| a̅c̅t̅i̅v̅a̅t̅i̅o̅n̅_38 (Activation) | (None, 260, 260, 32) | 0 | |
| batch_normalization_38 [0] [0] | | | |
| a̅c̅t̅i̅v̅a̅t̅i̅o̅n_41 (Activation) | (None, 260, 260, 64) | 0 | |
| batch_normalization_41[0] [0] | | | |
| b̅l̅o̅c̅k̅3̅5̅_̅5̅_mixed (Concatenate) | (None, 260, 260, 128 | 0 | activation_36[0] [0] |
| | | | activation_38[0] [0] |
| | | | activation_41[0] [0] |
| b̅l̅o̅c̅k̅3̅5̅_̅5̅_conv (Conv2D) | (None, 260, 260, 320 | 41280 | block35_5_mixed[0] [0] |
| b̅l̅o̅c̅k̅3̅5̅_̅5̅ (Lambda) | (None, 260, 260, 320 | 0 | block35_4_ac[0] [0] |
| | | | block35_5_conv[0] [0] |
| b̅l̅o̅c̅k̅3̅5̅_̅5̅_ac (Activation) | (None, 260, 260, 320 | 0 | block35_5[0] [0] |
| c̅o̅n̅v̅2̅d̅_̅4̅5̅ (Conv2D) | (None, 260, 260, 32) | 10240 | block35_5_ac[0] [0] |
| b̅a̅t̅c̅h̅_̅n̅o̅r̅malization_45 (BatchNo | (None, 260, 260, 32) | 96 | conv2d_45[0] [0] |
| a̅c̅t̅i̅v̅a̅t̅i̅o̅n_45 (Activation) | (None, 260, 260, 32) | 0 | |
| batch_normalization_45[0] [0] | | | |
| c̅o̅n̅v̅2̅d̅_̅4̅3̅ (Conv2D) | (None, 260, 260, 32) | 10240 | block35_5_ac[0] [0] |
| c̅o̅n̅v̅2̅d̅_̅4̅6̅ (Conv2D) | (None, 260, 260, 48) | 13824 | activation_45[0] [0] |
| b̅a̅t̅c̅h̅_̅n̅o̅r̅malization_43 (BatchNo | (None, 260, 260, 32) | 96 | conv2d_43 [0] [0] |
| b̅a̅t̅c̅h̅_̅n̅o̅r̅malization_46 (BatchNo | (None, 260, 260, 48) | 144 | conv2d_46[0] [0] |
| a̅c̅t̅i̅v̅a̅t̅i̅o̅n_43 (Activation) | (None, 260, 260, 32) | 0 | |
| batch_norma̅lization_43 [0] [0] | | | |
| a̅c̅t̅i̅v̅a̅t̅i̅o̅n_46 (Activation) | (None, 260, 260, 48) | 0 | |
| b̅a̅t̅c̅h̅_̅n̅o̅r̅malization_46[0] [0] | | | |
| c̅o̅n̅v̅2̅d̅_̅4̅2̅ (Conv2D) | (None, 260, 260, 32) | 10240 | block35_5_ac[0] [0] |
| c̅o̅n̅v̅2̅d̅_̅4̅4̅ (Conv2D) | (None, 260, 260, 32) | 9216 | activation_43 [0] [0] |
| c̅o̅n̅v̅2̅d̅_̅4̅7̅ (Conv2D) | (None, 260, 260, 64) | 27648 | activation_46 [0] [0] |
| b̅a̅t̅c̅h̅_̅n̅o̅r̅malization_42 (BatchNo | (None, 260, 260, 32) | 96 | conv2d_42[0] [0] |
| b̅a̅t̅c̅h̅_̅n̅o̅r̅malization_44 (BatchNo | (None, 260, 260, 32) | 9 6 | conv2d_44[0] [0] |
| b̅a̅t̅c̅h̅_̅n̅o̅r̅malization_47 (BatchNo | (None, 260, 260, 64) | 1 92 | conv2d_47 [0] [0] |
| a̅c̅t̅i̅v̅a̅t̅i̅o̅n_42 (Activation) | (None, 260, 260, 32) | 0 | |
| b̅a̅t̅c̅h̅_̅n̅o̅r̅malization_42[0] [0] | | | |
| a̅c̅t̅i̅v̅a̅t̅i̅o̅n_44 (Activation) | (None, 260, 260, 32) | 0 | |
| batch_norma̅lization_44[0] [0] | | | |
| a̅c̅t̅i̅v̅a̅t̅i̅o̅n_47 (Activation) | (None, 260, 260, 64) | 0 | |
| batch_normalization_47[0] [0] | | | |
| b̅l̅o̅c̅k̅3̅5̅_̅6̅_mixed (Concatenate) | (None, 260, 260, 128 | 0 | activation_42[0] [0] |
| | | | activation_44[0] [0] |
| | | | activation_47[0] [0] |
| b̅l̅o̅c̅k̅3̅5̅_̅6̅_conv (Conv2D) | (None, 260, 260, 320 | 4 41280 | block35_6_mixed[0] [0] |
| b̅l̅o̅c̅k̅3̅5̅_̅6̅ (Lambda) | (None, 260, 260, 320 | 0 | block35_5_ac[0] [0] |
| | | | block35_6_conv[0] [0] |
| b̅l̅o̅c̅k̅3̅5̅_̅6̅_ac (Activation) | (None, 260, 260, 320 | 0 | block35_6[0] [0] |
| c̅o̅n̅v̅2̅d̅_̅5̅1̅ (Conv2D) | (None, 260, 260, 32) | 1 10240 | block35_6_ac[0] [0] |
| b̅a̅t̅c̅h̅_̅n̅o̅r̅malization_51 (BatchNo | (None, 260, 260, 32) | 9 96 | conv2d_51[0] [0] |
| a̅c̅t̅i̅v̅a̅t̅i̅o̅n_51 (Activation) | (None, 260, 260, 32) | 0 | |
| b̅a̅t̅c̅h̅_̅n̅o̅r̅malization_51[0] [0] | | | |
| c̅o̅n̅v̅2̅d̅_̅4̅9̅ (Conv2D) | (None, 260, 260, 32) | 10240 | block35_6_ac[0] [0] |
| c̅o̅n̅v̅2̅d̅_̅5̅2̅ (Conv2D) | (None, 260, 260, 48) | 1 13824 | activation_51 [0] [0] |
| b̅a̅t̅c̅h̅_̅n̅o̅r̅malization_49 (BatchNo | (None, 260, 260, 32) | 9 96 | conv2d_49[0] [0] |
| b̅a̅t̅c̅h̅_̅n̅o̅r̅malization_52 (BatchNo | (None, 260, 260, 48) | 1 144 | conv2d_52[0] [0] |
| a̅c̅t̅i̅v̅a̅t̅i̅o̅n_49 (Activation) | (None, 260, 260, 32) | 0 | |
| batch_norma̅lization_49[0] [0] | | | |
| a̅c̅t̅i̅v̅a̅t̅i̅o̅n_52 (Activation) | (None, 260, 260, 48) | 0 | |
| batch_normalization_52[0] [0] | | | |
| c̅o̅n̅v̅2̅d̅_̅4̅8̅ (Conv2D) | (None, 260, 260, 32) | 1 10240 | block35_6_ac[0] [0] |
| c̅o̅n̅v̅2̅d̅_̅5̅0̅ (Conv2D) | (None, 260, 260, 32) | 9 9216 | activation_49 [0] [0] |
| c̅o̅n̅v̅2̅d̅_̅5̅3̅ (Conv2D) | (None, 260, 260, 64) | 2 27648 | activation_52[0] [0] |
| b̅a̅t̅c̅h̅_̅n̅o̅r̅malization_48 (BatchNo | (None, 260, 260, 32) | 96 | conv2d_48 [0] [0] |
| b̅a̅t̅c̅h̅_̅n̅o̅r̅malization_50 (BatchNo | (None, 260, 260, 32) | 96 | conv2d_50[0] [0] |
| b̅a̅t̅c̅h̅_̅n̅o̅r̅malization_53 (BatchNo | (None, 260, 260, 64) | 192 | conv2d_ 53[0] [0] |
| a̅c̅t̅i̅v̅a̅t̅i̅o̅n_48 (Activation) | (None, 260, 260, 32) | 0 | |
| batch_normalization_48[0] [0] | | | |
| a̅c̅t̅i̅v̅a̅t̅i̅o̅n_50 (Activation) | (None, 260, 260, 32) | 0 | |
| batch_normalization_50[0] [0] | | | |
| a̅c̅t̅i̅v̅a̅t̅i̅o̅n_53 (Activation) | (None, 260, 260, 64) | 0 | |
| batch_normalization_53[0] [0] | | | |
| b̅l̅o̅c̅k̅3̅5̅_̅7̅_mixed (Concatenate) | (None, 260, 260, 128 | 0 | activation_48 [0] [0] |
| | | | activation_50[0] [0] |
| | | | activation_53[0] [0] |
| b̅l̅o̅c̅k̅3̅5̅_̅7̅_conv (Conv2D) | (None, 260, 260, 320 | 41280 | block35_7_mixed[0] [0] |
| b̅l̅o̅c̅k̅3̅5̅_̅7̅ (Lambda) | (None, 260, 260, 320 | 0 | block35_6_ac[0] [0] |
| | | | block35_7_conv[0] [0] |
| b̅l̅o̅c̅k̅3̅5̅_̅7̅_ac (Activation) | (None, 260, 260, 320 | 0 | block35_7[0] [0] |
| c̅o̅n̅v̅2̅d̅_̅5̅7̅ (Conv2D) | (None, 260, 260, 32) | 10240 | block35_7_ac[0] [0] |
| b̅a̅t̅c̅h̅_̅n̅o̅r̅malization_57 (BatchNo | (None, 260, 260, 32) | 96 | conv2d_57[0] [0] |
| a̅c̅t̅i̅v̅a̅t̅i̅o̅n_57 (Activation) | (None, 260, 260, 32) | 0 | |
| batch_normalization_57[0] [0] | | | |
| c̅o̅n̅v̅2̅d̅_̅5̅5̅ (Conv2D) | (None, 260, 260, 32) | 10240 | block35_7_ac[0] [0] |
| c̅o̅n̅v̅2̅d̅_̅5̅8̅ (Conv2D) | (None, 260, 260, 48) | 13824 | activation_57 [0] [0] |
| b̅a̅t̅c̅h̅_̅n̅o̅r̅malization_55 (BatchNo | (None, 260, 260, 32) | 96 | conv2d_55[0] [0] |
| b̅a̅t̅c̅h̅_̅ n̅o̅r̅malization_58 (BatchNo | (None, 260, 260, 48) | 144 | conv2d_58[0] [0] |
| a̅c̅t̅i̅v̅a̅t̅i̅o̅n_55 (Activation) | (None, 260, 260, 32) | 0 | |
| b̅a̅t̅c̅h̅_̅n̅o̅r̅malization_55[0] [0] | | | |
| a̅c̅t̅i̅v̅a̅t̅i̅o̅n_58 (Activation) | (None, 260, 260, 48) | 0 | |
| b̅a̅t̅c̅h̅_̅n̅o̅r̅malization_58[0] [0] | | | |
| c̅o̅n̅v̅2̅d̅ 5̅4̅ (Conv2D) | (None, 260, 260, 32) | 10240 | block35_7_ac[0] [0] |
| c̅o̅n̅v̅2̅d̅_̅5̅6̅ (Conv2D) | (None, 260, 260, 32) | 9216 | activation_55 [0] [0] |
| c̅o̅n̅v̅2̅d̅_̅5̅9̅ (Conv2D) | (None, 260, 260, 64) | 27648 | activation_58 [0] [0] |
| b̅a̅t̅c̅h̅_̅n̅o̅r̅m̅alization_54 (BatchNo | (None, 260, 260, 32) | 96 | conv2d_54[0] [0] |
| b̅a̅t̅c̅h̅_̅ n̅o̅r̅m̅alization_56 (BatchNo | (None, 260, 260, 32) | 96 | conv2d_56[0] [0] |
| b̅a̅t̅c̅h̅_̅n̅o̅r̅m̅alization_59 (BatchNo | (None, 260, 260, 64) | 192 | conv2d_59[0] [0] |
| a̅c̅t̅i̅v̅a̅t̅i̅o̅n_54 (Activation) | (None, 260, 260, 32) | 0 | |
| batch_normalization_54[0] [0] | | | |
| a̅c̅t̅i̅v̅a̅t̅i̅o̅n_56 (Activation) | (None, 260, 260, 32) | 0 | |
| batch_normalization_56[0] [0] | | | |
| a̅c̅t̅i̅v̅a̅t̅i̅o̅n_59 (Activation) | (None, 260, 260, 64) | 0 | |
| batch_normalization_59[0] [0] | | | |
| b̅l̅o̅c̅k̅3̅5̅_̅8̅_mixed (Concatenate) | (None, 260, 260, 128 | 0 | activation_54 [0] [0] |
| | | | activation_56[0] [0] |
| | | | activation_59[0] [0] |
| b̅l̅o̅c̅k̅3̅5̅_̅8̅_conv (Conv2D) | (None, 260, 260, 320 | 41280 | block35_8_mixed[0] [0] |
| b̅l̅o̅c̅k̅3̅5̅_̅8̅ (Lambda) | (None, 260, 260, 320 | 0 | block35_7_ac[0] [0] |
| | | | block35_8_conv[0] [0] |
| b̅l̅o̅c̅k̅3̅5̅_̅8̅_ac (Activation) | (None, 260, 260, 320 | 0 | block35_8[0] [0] |
| c̅o̅n̅v̅2̅d̅_̅6̅3̅ (Conv2D) | (None, 260, 260, 32) | 10240 | block35_8_ac[0] [0] |
| b̅a̅t̅c̅h̅_̅n̅o̅r̅m̅alization_63 (BatchNo | (None, 260, 260, 32) | 96 | conv2d_63[0] [0] |
| a̅c̅t̅i̅v̅a̅t̅i̅o̅n_63 (Activation) | (None, 260, 260, 32) | 0 | |
| batch_normalization_63[0] [0] | | | |
| c̅o̅n̅v̅2̅d̅_̅6̅1̅ (Conv2D) | (None, 260, 260, 32) | 10240 | block35_8_ac[0] [0] |
| c̅o̅n̅v̅2̅d̅_̅6̅4̅ (Conv2D) | (None, 260, 260, 48) | 13824 | activation_63[0] [0] |
| b̅a̅t̅c̅h̅_̅n̅o̅r̅malization_61 (BatchNo | (None, 260, 260, 32) | 96 | conv2d_61[0] [0] |
| b̅a̅t̅c̅h̅_̅n̅o̅r̅malization_64 (BatchNo | (None, 260, 260, 48) | 144 | conv2d_64[0] [0] |
| a̅c̅t̅i̅v̅a̅t̅i̅o̅n_61 (Activation) | (None, 260, 260, 32) | 0 | |
| b̅a̅t̅c̅h̅_̅n̅o̅r̅malization_61[0] [0] | | | |
| a̅c̅t̅i̅v̅a̅t̅i̅o̅n_64 (Activation) | (None, 260, 260, 48) | 0 | |
| batch_normalization_64[0] [0] | | | |
| c̅o̅n̅v̅2̅d̅_̅6̅0̅ (Conv2D) | (None, 260, 260, 32) | 10240 | block35_8_ac[0] [0] |
| c̅o̅n̅v̅2̅d̅_̅6̅2̅ (Conv2D) | (None, 260, 260, 32) | 9216 | activation_61[0] [0] |
| c̅o̅n̅v̅2̅d̅_̅6̅5̅ (Conv2D) | (None, 260, 260, 64) | 27648 | activation_64[0] [0] |
| b̅a̅t̅c̅h̅_̅n̅o̅r̅malization_60 (BatchNo | (None, 260, 260, 32) | 96 | conv2d_60[0] [0] |
| b̅a̅t̅c̅h̅_̅n̅o̅r̅malization_62 (BatchNo | (None, 260, 260, 32) | 96 | conv2d_62[0] [0] |
| b̅a̅t̅c̅h̅_̅n̅o̅r̅malization_65 (BatchNo | (None, 260, 260, 64) | 192 | conv2d_65[0] [0] |
| a̅c̅t̅i̅v̅a̅t̅i̅o̅n_60 (Activation) | (None, 260, 260, 32) | 0 | |
| batch_normalization_60[0] [0] | | | |
| a̅c̅t̅i̅v̅a̅t̅i̅o̅n_62 (Activation) | (None, 260, 260, 32) | 0 | |
| batch_normalization_62 [0] [0] | | | |
| a̅c̅t̅i̅v̅a̅t̅i̅o̅n_65 (Activation) | (None, 260, 260, 64) | 0 | |
| batch_normalization_65[0] [0] | | | |
| b̅l̅o̅c̅k̅3̅5̅_̅9̅_mixed (Concatenate) | (None, 260, 260, 128 | 0 | activation_60 [0] [0] |
| | | | activation_62[0] [0] |
| | | | activation_65[0] [0] |
| b̅l̅o̅c̅k̅3̅5̅_̅9̅_conv (Conv2D) | (None, 260, 260, 320 | 41280 | block35_9_mixed[0] [0] |
| b̅l̅o̅c̅k̅3̅5̅_̅9̅ (Lambda) | (None, 260, 260, 320 | 0 | block35_8_ac[0] [0] |
| | | | block35_9_conv[0] [0] |
| b̅l̅o̅c̅k̅3̅5̅_̅9̅_ac (Activation) | (None, 260, 260, 320 | 0 | block35_9[0] [0] |
| c̅o̅n̅v̅2̅d̅_̅6̅9̅ (Conv2D) | (None, 260, 260, 32) | 10240 | block35_9_ac[0] [0] |
| b̅a̅t̅c̅h̅_̅n̅o̅r̅malization_69 (BatchNo | (None, 260, 260, 32) | 96 | conv2d_69[0] [0] |
| a̅c̅t̅i̅v̅a̅t̅i̅o̅n_69 (Activation) | (None, 260, 260, 32) | 0 | |
| batch_normalization_69[0] [0] | | | |
| c̅o̅n̅v̅2̅d̅_̅6̅7̅ (Conv2D) | (None, 260, 260, 32) | 10240 | block35_9_ac[0] [0] |
| c̅o̅n̅v̅2̅d̅_̅7̅0̅ (Conv2D) | (None, 260, 260, 48) | 13824 | activation_69[0] [0] |
| b̅a̅t̅c̅h̅_̅n̅o̅r̅malization_67 (BatchNo | (None, 260, 260, 32) | 96 | conv2d_67 [0] [0] |
| b̅a̅t̅c̅h̅_̅n̅o̅r̅malization_70 (BatchNo | (None, 260, 260, 48) | 144 | conv2d_70[0] [0] |
| a̅c̅t̅i̅v̅a̅t̅i̅o̅n_67 (Activation) | (None, 260, 260, 32) | 0 | |
| batch_normalization_67[0] [0] | | | |
| a̅c̅t̅i̅v̅a̅t̅i̅o̅n_70 (Activation) | (None, 260, 260, 48) | 0 | |
| batch_normalization_70[0] [0] | | | |
| c̅o̅n̅v̅2̅d̅_̅6̅6̅ (Conv2D) | (None, 260, 260, 32) | 10240 | block35_9_ac[0] [0] |
| c̅o̅n̅v̅2̅d̅_̅6̅8̅ (Conv2D) | (None, 260, 260, 32) | 9216 | activation_67 [0] [0] |
| c̅o̅n̅v̅2̅d̅_̅7̅1̅ (Conv2D) | (None, 260, 260, 64) | 27648 | activation_70 [0] [0] |
| b̅a̅t̅c̅h̅_̅n̅o̅r̅malization_66 (BatchNo | (None, 260, 260, 32) | 96 | conv2d_66[0] [0] |
| b̅a̅t̅c̅h̅_̅n̅o̅r̅malization_68 (BatchNo | (None, 260, 260, 32) | 96 | conv2d_68 [0] [0] |
| b̅a̅t̅c̅h̅_̅n̅o̅r̅malization_71 (BatchNo | (None, 260, 260, 64) | 192 | conv2d_71 [0] [0] |
| a̅c̅t̅i̅v̅a̅t̅i̅o̅n_66 (Activation) | (None, 260, 260, 32) | 0 | |
| batch_normalization_66[0] [0] | | | |
| a̅c̅t̅i̅v̅a̅t̅i̅o̅n_68 (Activation) | (None, 260, 260, 32) | 0 | |
| batch_normalization_68[0] [0] | | | |
| a̅c̅t̅i̅v̅a̅t̅i̅o̅n_71 (Activation) | (None, 260, 260, 64) | 0 | |
| batch_normalization_71[0] [0] | | | |
| b̅l̅o̅c̅k̅3̅5̅_̅1̅0̅_mixed (Concatenate) | (None, 260, 260, 128 | 0 | activation_66[0] [0] |
| | | | activation_68 [0] [0] |
| | | | activation_71[0] [0] |
| b̅l̅o̅c̅k̅3̅5̅_̅1̅0̅_conv (Conv2D) | (None, 260, 260, 320 | 41280 | block35_10_mixed[0] [0] |
| b̅l̅o̅c̅k̅3̅5̅_̅1̅0̅ (Lambda) | (None, 260, 260, 320 | 0 | block35_9_ac[0] [0] |
| | | | block35_10_conv[0] [0] |
| b̅l̅o̅c̅k̅3̅5̅_̅1̅0̅_ac (Activation) | (None, 260, 260, 320 | 0 | block35_10[0] [0] |
| c̅o̅n̅v̅2̅d̅_̅7̅3̅ (Conv2D) | (None, 260, 260, 256 | 81920 | block35_10_ac[0] [0] |
| b̅a̅t̅c̅h̅_̅n̅o̅r̅malization_73 (BatchNo | (None, 260, 260, 256 | 768 | conv2d_73[0] [0] |
| a̅c̅t̅i̅v̅a̅t̅i̅o̅n_73 (Activation) | (None, 260, 260, 256 | 0 | |
| batch_normalization_73[0] [0] | | | |
| c̅o̅n̅v̅2̅d̅_̅7̅4̅ (Conv2D) | (None, 260, 260, 256 | 589824 | activation_73 [0] [0] |
| b̅a̅t̅c̅h̅_̅n̅o̅r̅malization_74 (BatchNo | (None, 260, 260, 256 | 768 | conv2d_74 [0] [0] |
| a̅c̅t̅i̅v̅a̅t̅i̅o̅n_74 (Activation) | (None, 260, 260, 256 | 0 | |
| batch_normalization_74[0] [0] | | | |
| c̅o̅n̅v̅2̅d̅_̅7̅2̅ (Conv2D) | (None, 129, 129, 384 | 1105920 | block35_10_ac[0] [0] |
| c̅o̅n̅v̅2̅d̅_̅7̅5̅ (Conv2D) | (None, 129, 129, 384 | 884736 | activation_74 [0] [0] |
| b̅a̅t̅c̅h̅_̅n̅o̅r̅malization_72 (BatchNo | (None, 129, 129, 384 | 1152 | conv2d_72[0] [0] |
| b̅a̅t̅c̅h̅_̅n̅o̅r̅malization_75 (BatchNo | (None, 129, 129, 384 | 1152 | conv2d_75[0] [0] |
| a̅c̅t̅i̅v̅a̅t̅i̅o̅n_72 (Activation) | (None, 129, 129, 384 | 0 | |
| batch_normalization_72[0] [0] | | | |
| a̅c̅t̅i̅v̅a̅t̅i̅o̅n_75 (Activation) | (None, 129, 129, 384 | 0 | |
| batch_normalization_75[0][0] | | | |
| m̅a̅x̅_̅p̅o̅o̅l̅i̅n̅g2d_2 (MaxPooling2D) | (None, 129, 129, 320 | 0 | block35_10_ac[0] [0] |
| m̅i̅x̅e̅d̅_̅6̅a̅ (Concatenate) | (None, 129, 129, 108 | 0 | activation_72 [0] [0] |
| | | | activation_75[0] [0] |
| | | | max_poolin 2d_2 [0] [0] |
| c̅o̅n̅v̅2̅d̅_̅7̅7̅ (Conv2D) | (None, 129, 129, 128 | 139264 | mixed_6a[0] [0] |
| b̅a̅t̅c̅h̅_̅n̅o̅r̅malization_77 (BatchNo | (None, 129, 129, 128 | 384 | conv2d_77 [0] [0] |
| a̅c̅t̅i̅v̅a̅t̅i̅o̅n_77 (Activation) | (None, 129, 129, 128 | 0 | |
| batch_normalization_77[0] [0] | | | |
| c̅o̅n̅v̅2̅d̅_̅7̅8̅ (Conv2D) | (None, 129, 129, 160 | 143360 | activation_77 [0] [0] |
| b̅a̅t̅c̅h̅_̅n̅o̅r̅malization_78 (BatchNo | (None, 129, 129, 160 | 480 | conv2d_78[0] [0] |
| a̅c̅t̅i̅v̅a̅t̅i̅o̅n_78 (Activation) | (None, 129, 129, 160 | 0 | |
| batch_normalization_78[0] [0] | | | |
| c̅o̅n̅v̅2̅d̅_̅7̅6̅ (Conv2D) | (None, 129, 129, 192 | 208896 | mixed_6a[0] [0] |
| c̅o̅n̅v̅2̅d̅_̅7̅9̅ (Conv2D) | (None, 129, 129, 192 | 215040 | activation_78 [0] [0] |
| b̅a̅t̅c̅h̅_̅n̅o̅r̅malization_76 (BatchNo | (None, 129, 129, 192 | 576 | conv2d_76[0] [0] |
| b̅a̅t̅c̅h̅_̅n̅o̅r̅malization_79 (BatchNo | (None, 129, 129, 192 | 576 | conv2d_79[0] [0] |
| a̅c̅t̅i̅v̅a̅t̅i̅o̅n_76 (Activation) | (None, 129, 129, 192 | 0 | |
| batch_normalization_76[0] [0] | | | |
| a̅c̅t̅i̅v̅a̅t̅i̅o̅n_79 (Activation) | (None, 129, 129, 192 | 0 | |
| batch_normalization_79 [0] [0] | | | |
| b̅l̅o̅c̅k̅1̅7̅_̅1̅_mixed (Concatenate) | (None, 129, 129, 384 | 0 | activation_76 [0] [0] |
| | | | activation_79[0] [0] |
| b̅l̅o̅c̅k̅1̅7̅_̅1̅_conv (Conv2D) | (None, 129, 129, 108 | 418880 | block17_1_mixed[0] [0] |
| b̅l̅o̅c̅k̅1̅7̅_̅1̅ (Lambda) | (None, 129, 129, 108 | 0 | mixed_6a[0] [0] |
| | | | block17_1_conv[0] [0] |
| b̅l̅o̅c̅k̅1̅7̅_̅1̅_ac (Activation) | (None, 129, 129, 108 | 0 | block17_1[0] [0] |
| c̅o̅n̅v̅2̅d̅_̅8̅1̅ (Conv2D) | (None, 129, 129, 128 | 139264 | block17_1_ac[0] [0] |
| b̅a̅t̅c̅h̅_̅n̅o̅r̅malization_81 (BatchNo | (None, 129, 129, 128 | 384 | conv2d_81 [0] [0] |
| a̅c̅t̅i̅v̅a̅t̅i̅o̅n_81 (Activation) | (None, 129, 129, 128 | 0 | |
| batch_normalization_81[0] [0] | | | |
| c̅o̅n̅v̅2̅d̅_̅8̅2̅ (Conv2D) | (None, 129, 129, 160 | 143360 | activation_81 [0] [0] |
| b̅a̅t̅c̅h̅_̅n̅o̅r̅malization_82 (BatchNo | (None, 129, 129, 160 | 480 | conv2d_82 [0] [0] |
| a̅c̅t̅i̅v̅a̅t̅i̅o̅n_82 (Activation) | (None, 129, 129, 160 | 0 | |
| batch_normalization_82[0][0] | | | |
| c̅o̅n̅v̅2̅d̅_̅8̅0̅ (Conv2D) | (None, 129, 129, 192 | 208896 | block17_1_ac[0] [0] |
| c̅o̅n̅v̅2̅d̅_̅8̅3̅ (Conv2D) | (None, 129, 129, 192 | 215040 | activation_82 [0] [0] |
| b̅a̅t̅c̅h̅_̅n̅o̅r̅malization_80 (BatchNo | (None, 129, 129, 192 | 576 | conv2d_80[0] [0] |
| b̅a̅t̅c̅h̅_̅n̅o̅r̅malization_83 (BatchNo | (None, 129, 129, 192 | 576 | conv2d_83[0] [0] |
| a̅c̅t̅i̅v̅a̅t̅i̅o̅n_80 (Activation) | (None, 129, 129, 192 | 0 | |
| batch__normalization_80[0] [0] | | | |
| a̅c̅t̅i̅v̅a̅t̅i̅o̅n_83 (Activation) | (None, 129, 129, 192 | 0 | |
| batch_normalization_83[0] [0] | | | |
| b̅l̅o̅c̅k̅1̅7̅_̅2̅_mixed (Concatenate) | (None, 129, 129, 384 | 0 | activation_80 [0] [0] |
| | | | activation_83[0] [0] |
| b̅l̅o̅c̅k̅1̅7̅_̅2̅_conv (Conv2D) | (None, 129, 129, 108 | 418880 | block17_2_mixed[0] [0] |
| block17_2 (Lambda) | (None, 129, 129, 108 | 0 | block17_1_ac[0] [0] |
| | | | block17_2_conv[0] [0] |
| b̅l̅o̅c̅k̅1̅7̅_̅2̅_ac (Activation) | (None, 129, 129, 108 | 0 | block17_2[0] [0] |
| c̅o̅n̅v̅2̅d̅_̅8̅5̅ (Conv2D) | (None, 129, 129, 128 | 139264 | block17_2_ac[0] [0] |
| b̅a̅t̅c̅h̅_̅n̅o̅r̅malization_85 (BatchNo | (None, 129, 129, 128 | 384 | conv2d_85[0] [0] |
| a̅c̅t̅i̅v̅a̅t̅i̅o̅n_85 (Activation) | (None, 129, 129, 128 | 0 | |
| batch_normalization_85[0] [0] | | | |
| c̅o̅n̅v̅2̅d̅_̅8̅6̅ (Conv2D) | (None, 129, 129, 160 | 143360 | activation_85[0][0] |
| b̅a̅t̅c̅h̅_̅n̅o̅r̅malization_86 (BatchNo | (None, 129, 129, 160 | 480 | conv2d_86[0][0] |
| a̅c̅t̅i̅v̅a̅t̅i̅o̅n_86 (Activation) | (None, 129, 129, 160 | 0 | |
| batch_normalization_86[0] [0] | | | |
| c̅o̅n̅v̅2̅d̅_̅8̅4̅ (Conv2D) | (None, 129, 129, 192 | 208896 | block17_2_ac[0][0] |
| c̅o̅n̅v̅2̅d̅_̅8̅7̅ (Conv2D) | (None, 129, 129, 192 | 215040 | activation_86[0][0] |
| b̅a̅t̅c̅h̅_̅n̅o̅r̅malization_84 (BatchNo | (None, 129, 129, 192 | 576 | conv2d_84[0][0] |
| b̅a̅t̅c̅h̅_̅n̅o̅r̅malization_87 (BatchNo | (None, 129, 129, 192 | 576 | conv2d_87[0][0] |
| a̅c̅t̅i̅v̅a̅t̅i̅o̅n_84 (Activation) | (None, 129, 129, 192 | 0 | |
| batch_normalization_84[0] [0] | | | |
| a̅c̅t̅i̅v̅a̅t̅i̅o̅n_87 (Activation) | (None, 129, 129, 192 | 0 | |
| batch_normalization_87[0] [0] | | | |
| b̅l̅o̅c̅k̅1̅7̅_̅3̅_mixed (Concatenate) | (None, 129, 129, 384 | 0 | activation_84[0][0] |
| | | | activation_87[0][0] |
| b̅l̅o̅c̅k̅1̅7̅_̅3̅_conv (Conv2D) | (None, 129, 129, 108 | 418880 | block17_3_mixed[0][0] |
| b̅l̅o̅c̅k̅1̅7̅_̅3̅ (Lambda) | (None, 129, 129, 108 | 0 | block17_2_ac[0] [0] |
| | | | block17_3_conv[0][0] |
| b̅l̅o̅c̅k̅1̅7̅_̅3̅_ac (Activation) | (None, 129, 129, 108 | 0 | block17_3[0][0] |
| c̅o̅n̅v̅2̅d̅_̅8̅9̅ (Conv2D) | (None, 129, 129, 128 | 139264 | block17_3_ac[0][0] |
| b̅a̅t̅c̅h̅_̅n̅o̅r̅malization_89 (BatchNo | (None, 129, 129, 128 | 384 | conv2d_89[0][0] |
| a̅c̅t̅i̅v̅a̅t̅i̅o̅n_89 (Activation) | (None, 129, 129, 128 | 0 | |
| batch_normalization_89[0] [0] | | | |
| c̅o̅n̅v̅2̅d̅_̅9̅0̅ (Conv2D) | (None, 129, 129, 160 | 143360 | activation_89[0][0] |
| b̅a̅t̅c̅h̅_̅n̅o̅r̅malization_90 (BatchNo | (None, 129, 129, 160 | 480 | conv2d_90[0][0] |
| a̅c̅t̅i̅v̅a̅t̅i̅o̅n_90 (Activation) | (None, 129, 129, 160 | 0 | |
| batch_normalization_90[0] [0] | | | |
| c̅o̅n̅v̅2̅d̅_̅8̅8̅ (Conv2D) | (None, 129, 129, 192 | 208896 | block17_3 _ac[0][0] |
| c̅o̅n̅v̅2̅d̅_̅9̅1̅ (Conv2D) | (None, 129, 129, 192 | 215040 | activation_90 [0] [0] |
| b̅a̅t̅c̅h̅_̅n̅o̅r̅malization_88 (BatchNo | (None, 129, 129, 192 | 576 | conv2d_88[0] [0] |
| b̅a̅t̅c̅h̅_̅n̅o̅r̅malization_91 (BatchNo | (None, 129, 129, 192 | 576 | conv2d_ 91[0] [0] |
| a̅c̅t̅i̅v̅a̅t̅i̅o̅n_88 (Activation) | (None, 129, 129, 192 | 0 | |
| batch_normalization_88 [0] [0] | | | |
| a̅c̅t̅i̅v̅a̅t̅i̅o̅n_91 (Activation) | (None, 129, 129, 192 | 0 | |
| batch_normalization_ 91[0] [0] | | | |
| b̅l̅o̅c̅k̅1̅7̅_̅4̅_mixed (Concatenate) | (None, 129, 129, 384 | 0 | activation_88 [0] [0] |
| | | | activation_91[0] [0] |
| b̅l̅o̅c̅k̅1̅7̅_̅4̅_conv (Conv2D) | (None, 129, 129, 108 | 418880 | block17_4_mixed[0] [0] |
| b̅l̅o̅c̅k̅1̅7̅_̅4̅ (Lambda) | (None, 129, 129, 108 | 0 | block17_3_ac[0] [0] |
| | | | block17_4_conv[0] [0] |
| b̅l̅o̅c̅k̅1̅7̅_̅4̅_ac (Activation) | (None, 129, 129, 108 | 0 | block17_4[0] [0] |
| c̅o̅n̅v̅2̅d̅_̅9̅3̅ (Conv2D) | (None, 129, 129, 128 | 139264 | block17_4_ac[0] [0] |
| b̅a̅t̅c̅h̅_̅n̅o̅r̅malization_93 (BatchNo | (None, 129, 129, 128 | 384 | conv2d_93[0] [0] |
| a̅c̅t̅i̅v̅a̅t̅i̅o̅n_93 (Activation) | (None, 129, 129, 128 | 0 | |
| batch_normalization_93[0] [0] | | | |
| c̅o̅n̅v̅2̅d̅_̅9̅4̅ (Conv2D) | (None, 129, 129, 160 | 143360 | activation_93 [0] [0] |
| b̅a̅t̅c̅h̅_̅n̅o̅r̅malization_94 (BatchNo | (None, 129, 129, 160 | 480 | conv2d_94[0] [0] |
| a̅c̅t̅i̅v̅a̅t̅i̅o̅n_94 (Activation) | (None, 129, 129, 160 | 0 | |
| batch_normalization_94[0] [0] | | | |
| c̅o̅n̅v̅2̅d̅_̅9̅2̅ (Conv2D) | (None, 129, 129, 192 | 208896 | block17_4_ac[0] [0] |
| c̅o̅n̅v̅2̅d̅_̅9̅5̅ (Conv2D) | (None, 129, 129, 192 | 215040 | activation_94 [0] [0] |
| b̅a̅t̅c̅h̅_̅n̅o̅r̅malization_92 (BatchNo | (None, 129, 129, 192 | 576 | conv2d_92[0] [0] |
| b̅a̅t̅c̅h̅_̅n̅o̅r̅malization_95 (BatchNo | (None, 129, 129, 192 | 576 | conv2d_95[0] [0] |
| a̅c̅t̅i̅v̅a̅t̅i̅o̅n_92 (Activation) | (None, 129, 129, 192 | 0 | |
| batch_normalization_92[0] [0] | | | |
| a̅c̅t̅i̅v̅a̅t̅i̅o̅n_95 (Activation) | (None, 129, 129, 192 | 0 | |
| batch_normalization_95[0] [0] | | | |
| b̅l̅o̅c̅k̅1̅7̅_̅5̅_mixed (Concatenate) | (None, 129, 129, 384 | 0 | activation_92 [0] [0] |
| | | | activation_95[0] [0] |
| b̅l̅o̅c̅k̅1̅7̅_̅5̅_conv (Conv2D) | (None, 129, 129, 108 | 418880 | block17_5_mixed[0] [0] |
| b̅l̅o̅c̅k̅1̅7̅_̅5̅ (Lambda) | (None, 129, 129, 108 | 0 | block17_4_ac[0] [0] |
| | | | block17_5_conv[0] [0] |
| b̅l̅o̅c̅k̅1̅7̅_̅5̅_ac (Activation) | (None, 129, 129, 108 | 0 | block17_5[0] [0] |
| c̅o̅n̅v̅2̅d̅_̅9̅7̅ (Conv2D) | (None, 129, 129, 128 | 139264 | block17_5_ac[0] [0] |
| b̅a̅t̅c̅h̅_̅n̅o̅r̅malization_97 (BatchNo | (None, 129, 129, 128 | 384 | conv2d_97 [0] [0] |
| a̅c̅t̅i̅v̅a̅t̅i̅o̅n_97 (Activation) | (None, 129, 129, 128 | 0 | |
| batch_normalization_97[0] [0] | | | |
| c̅o̅n̅v̅2̅d̅_̅9̅8̅ (Conv2D) | (None, 129, 129, 160 | 143360 | activation_97 [0] [0] |
| b̅a̅t̅c̅h̅_̅n̅o̅r̅malization_98 (BatchNo | (None, 129, 129, 160 | 480 | conv2d_98 [0] [0] |
| a̅c̅t̅i̅v̅a̅t̅i̅o̅n_98 (Activation) | (None, 129, 129, 160 | 0 | |
| batch_normalization_98 [0] [0] | | | |
| c̅o̅n̅v̅2̅d̅_̅9̅6̅ (Conv2D) | (None, 129, 129, 192 | 208896 | block17_5_ac[0] [0] |
| c̅o̅n̅v̅2̅d̅_̅9̅9̅ (Conv2D) | (None, 129, 129, 192 | 215040 | activation_98 [0] [0] |
| b̅a̅t̅c̅h̅_̅n̅o̅r̅malization_96 (BatchNo | (None, 129, 129, 192 | 576 | conv2d_96[0] [0] |
| b̅a̅t̅c̅h̅_̅n̅o̅r̅malization_99 (BatchNo | (None, 129, 129, 192 | 576 | conv2d_99[0] [0] |
| a̅c̅t̅i̅v̅a̅t̅i̅o̅n_96 (Activation) | (None, 129, 129, 192 | 0 | |
| batch_norma̅lization_96[0] [0] | | | |
| a̅c̅t̅i̅v̅a̅t̅i̅o̅n_99 (Activation) | (None, 129, 129, 192 | 0 | |
| batch_normalization_99[0] [0] | | | |
| b̅l̅o̅c̅k̅1̅7̅_̅6̅_mixed (Concatenate) | (None, 129, 129, 384 | 0 | activation_96 [0] [0] |
| | | | activation_99[0] [0] |
| b̅l̅o̅c̅k̅1̅7̅_̅6̅_conv (Conv2D) | (None, 129, 129, 108 | 418880 | block17_6_mixed[0] [0] |
| b̅l̅o̅c̅k̅1̅7̅_̅6̅ (Lambda) | (None, 129, 129, 108 | 0 | block17_5_ac[0] [0] |
| | | | block17_6_conv[0] [0] |
| b̅l̅o̅c̅k̅1̅7̅_̅6̅_ac (Activation) | (None, 129, 129, 108 | 0 | block17_6[0] [0] |
| c̅o̅n̅v̅2̅d̅_̅1̅0̅1 (Conv2D) | (None, 129, 129, 128 | 139264 | block17_6_ac[0] [0] |
| b̅a̅t̅c̅h̅_̅n̅o̅r̅malization_101 (BatchN | (None, 129, 129, 128 | 384 | conv2d_101[0][0] |
| a̅c̅t̅i̅v̅a̅t̅i̅o̅n_101 (Activation) | (None, 129, 129, 128 | 0 | |
| batch_normalization_101[0][0] | | | |
| c̅o̅n̅v̅2̅d̅_̅1̅0̅2̅ (Conv2D) | (None, 129, 129, 160 | 143360 | activation_101[0][0] |
| b̅a̅t̅c̅h̅_̅n̅o̅r̅malization_102 (BatchN | (None, 129, 129, 160 | 480 | conv2d_102[0][0] |
| a̅c̅t̅i̅v̅a̅t̅i̅o̅n_102 (Activation) | (None, 129, 129, 160 | 0 | |
| batch_normalization_102[0][0] | | | |
| c̅o̅n̅v̅2̅d̅_̅1̅0̅0 (Conv2D) | (None, 129, 129, 192 | 208896 | block17_6_ac[0][0] |
| c̅o̅n̅v̅2̅d̅_̅1̅0̅3 (Conv2D) | (None, 129, 129, 192 | 215040 | activation_102[0][0] |
| b̅a̅t̅c̅h̅_̅n̅o̅r̅malization_100 (BatchN | (None, 129, 129, 192 | 576 | conv2d_100[0][0] |
| b̅a̅t̅c̅h̅_̅n̅o̅r̅malization_103 (BatchN | (None, 129, 129, 192 | 576 | conv2d_103[0][0] |
| a̅c̅t̅i̅v̅a̅t̅i̅o̅n_100 (Activation) | (None, 129, 129, 192 | 0 | |
| batch_normalization_100[0][0] | | | |
| a̅c̅t̅i̅v̅a̅t̅i̅o̅n_103 (Activation) | (None, 129, 129, 192 | 0 | |
| batch_normalization_103[0][0] | | | |
| b̅l̅o̅c̅k̅1̅7̅_̅7̅_mixed (Concatenate) | (None, 129, 129, 384 | 0 | activation_100[0][0] |
| | | | activation_103[0][0] |
| b̅l̅o̅c̅k̅1̅7̅_̅7̅_conv (Conv2D) | (None, 129, 129, 108 | 418880 | block17_7_mixed[0][0] |
| b̅l̅o̅c̅k̅1̅7̅_̅7̅ (Lambda) | (None, 129, 129, 108 | 0 | block17_6_ac[0][0] |
| | | | block17_7_conv[0][0] |
| b̅l̅o̅c̅k̅1̅7̅_̅7̅_ac (Activation) | (None, 129, 129, 108 | 0 | block17_7[0][0] |
| c̅o̅n̅v̅2̅d̅_̅1̅0̅5 (Conv2D) | (None, 129, 129, 128 | 139264 | block17_7_ac[0][0] |
| b̅a̅t̅c̅h̅_̅n̅o̅r̅malization_105 (BatchN | (None, 129, 129, 128 | 384 | conv2d_105[0][0] |
| a̅c̅t̅i̅v̅a̅t̅i̅o̅n_105 (Activation) | (None, 129, 129, 128 | 0 | |
| b̅a̅t̅c̅h̅_̅n̅o̅r̅malization_105[0][0] | | | |
| c̅o̅n̅v̅2̅d̅_̅1̅0̅6 (Conv2D) | (None, 129, 129, 160 | 143360 | activation_105[0][0] |
| batch_normalization_106 (BatchN | (None, 129, 129, 160 | 480 | conv2d_106[0][0] |
| a̅c̅t̅i̅v̅a̅t̅i̅o̅n_106 (Activation) | (None, 129, 129, 160 | 0 | |
| b̅a̅t̅c̅h̅_̅n̅o̅r̅malization_106[0][0] | | | |
| c̅o̅n̅v̅2̅d̅_̅1̅0̅4 (Conv2D) | (None, 129, 129, 192 | 208896 | block17_7_ac[0][0] |
| c̅o̅n̅v̅2̅d̅_̅1̅0̅7 (Conv2D) | (None, 129, 129, 192 | 215040 | activation_106[0][0] |
| b̅a̅t̅c̅h̅_̅n̅o̅r̅malization_104 (BatchN | (None, 129, 129, 192 | 576 | conv2d_104[0][0] |
| b̅a̅t̅c̅h̅_̅n̅o̅r̅malization_107 (BatchN | (None, 129, 129, 192 | 576 | conv2d_107[0][0] |
| a̅c̅t̅i̅v̅a̅t̅i̅o̅n_104 (Activation) | (None, 129, 129, 192 | 0 | |
| batch_normalization_104[0][0] | | | |
| a̅c̅t̅i̅v̅a̅t̅i̅o̅n_107 (Activation) | (None, 129, 129, 192 | 0 | |
| b̅a̅t̅c̅h̅_̅n̅o̅r̅malization_107[0][0] | | | |
| b̅l̅o̅c̅k̅1̅7̅_̅8̅_mixed (Concatenate) | (None, 129, 129, 384 | 0 | activation_104[0][0] |
| | | | activation_107[0][0] |
| b̅l̅o̅c̅k̅1̅7̅_̅8̅_conv (Conv2D) | (None, 129, 129, 108 | 418880 | block17_8_mixed[0][0] |
| b̅l̅o̅c̅k̅1̅7̅_̅8̅ (Lambda) | (None, 129, 129, 108 | 0 | block17_7_ac[0][0] |
| | | | block17_8_conv[0][0] |
| b̅l̅o̅c̅k̅1̅7̅_̅8̅_ac (Activation) | (None, 129, 129, 108 | 0 | block17_8[0][0] |
| c̅o̅n̅v̅2̅d̅_̅1̅0̅9 (Conv2D) | (None, 129, 129, 128 | 139264 | block17_8_ac[0][0] |
| b̅a̅t̅c̅h̅_̅n̅o̅r̅malization_109 (BatchN | (None, 129, 129, 128 | 384 | conv2d_109[0][0] |
| a̅c̅t̅i̅v̅a̅t̅i̅o̅n_109 (Activation) | (None, 129, 129, 128 | 0 | |
| batch_normalization_109[0][0] | | | |
| c̅o̅n̅v̅2̅d̅_̅1̅1̅0 (Conv2D) | (None, 129, 129, 160 | 143360 | activation_109[0][0] |
| b̅a̅t̅c̅h̅_̅n̅o̅r̅malization_110 (BatchN | (None, 129, 129, 160 | 480 | conv2d_110[0][0] |
| a̅c̅t̅i̅v̅a̅t̅i̅o̅n_110 (Activation) | (None, 129, 129, 160 | 0 | |
| batch_normalization_110[0][0] | | | |
| c̅o̅n̅v̅2̅d̅_̅1̅0̅8 (Conv2D) | (None, 129, 129, 192 | 208896 | block17_8_ac[0][0] |
| c̅o̅n̅v̅2̅d̅_̅1̅1̅1 (Conv2D) | (None, 129, 129, 192 | 215040 | activation_110[0][0] |
| b̅a̅t̅c̅h̅_̅n̅o̅r̅malization_108 (BatchN | (None, 129, 129, 192 | 576 | conv2d_108[0][0] |
| b̅a̅t̅c̅h̅_̅n̅o̅r̅malization_111 (BatchN | (None, 129, 129, 192 | 576 | conv2d_111[0][0] |
| a̅c̅t̅i̅v̅a̅t̅i̅o̅n_108 (Activation) | (None, 129, 129, 192 | 0 | |
| batch_normalization_108[0][0] | | | |
| a̅c̅t̅i̅v̅a̅t̅i̅o̅n_111 (Activation) | (None, 129, 129, 192 | 0 | |
| batch_normalization_111[0][0] | | | |
| b̅l̅o̅c̅k̅1̅7̅_̅9̅_mixed (Concatenate) | (None, 129, 129, 384 | 0 | activation_108[0][0] |
| | | | activation_111[0][0] |
| b̅l̅o̅c̅k̅1̅7̅_̅9̅_conv (Conv2D) | (None, 129, 129, 108 | 418880 | block17_9_mixed[0][0] |
| b̅l̅o̅c̅k̅1̅7̅_̅9̅ (Lambda) | (None, 129, 129, 108 | 0 | block17_8_ac[0][0] |
| | | | block17_9_conv[0][0] |
| b̅l̅o̅c̅k̅1̅7̅_̅9̅_ac (Activation) | (None, 129, 129, 108 | 0 | block17_9[0][0] |
| c̅o̅n̅v̅2̅d̅_̅1̅1̅3 (Conv2D) | (None, 129, 129, 128 | 139264 | block17_9_ac[0][0] |
| b̅a̅t̅c̅h̅_̅n̅o̅r̅malization_113 (BatchN | (None, 129, 129, 128 | 384 | conv2d_113[0][0] |
| a̅c̅t̅i̅v̅a̅t̅i̅o̅n_113 (Activation) | (None, 129, 129, 128 | 0 | |
| batch_normalization_113[0][0] | | | |
| c̅o̅n̅v̅2̅d̅_̅1̅1̅4 (Conv2D) | (None, 129, 129, 160 | 143360 | activation_113[0][0] |
| b̅a̅t̅c̅h̅_̅n̅o̅r̅malization_114 (BatchN | (None, 129, 129, 160 | 480 | conv2d_114[0][0] |
| a̅c̅t̅i̅v̅a̅t̅i̅o̅n_114 (Activation) | (None, 129, 129, 160 | 0 | |
| batch_normalization_114[0][0] | | | |
| c̅o̅n̅v̅2̅d̅_̅1̅1̅2 (Conv2D) | (None, 129, 129, 192 | 208896 | block17_9_ac[0][0] |
| c̅o̅n̅v̅2̅d̅_̅1̅1̅5 (Conv2D) | (None, 129, 129, 192 | 215040 | activation_114[0][0] |
| b̅a̅t̅c̅h̅_̅n̅o̅r̅malization_112 (BatchN | (None, 129, 129, 192 | 576 | conv2d_112[0][0] |
| b̅a̅t̅c̅h̅_̅n̅o̅r̅malization_115 (BatchN | (None, 129, 129, 192 | 576 | conv2d_115[0][0] |
| a̅c̅t̅i̅v̅a̅t̅i̅o̅n_112 (Activation) | (None, 129, 129, 192 | 0 | |
| b̅a̅t̅c̅h̅_̅n̅o̅r̅malization_112[0][0] | | | |
| a̅c̅t̅i̅v̅a̅t̅i̅o̅n_115 (Activation) | (None, 129, 129, 192 | 0 | |
| batch_normalization_115[0][0] | | | |
| b̅l̅o̅c̅k̅1̅7̅_̅1̅0̅_mixed (Concatenate) | (None, 129, 129, 384 | 0 | activation_112[0][0] |
| | | | activation_115[0][0] |
| b̅l̅o̅c̅k̅1̅7̅_̅1̅0̅_conv (Conv2D) | (None, 129, 129, 108 | 418880 | block17_10_mixed[0][0] |
| b̅l̅o̅c̅k̅1̅7̅_̅1̅0̅ (Lambda) | (None, 129, 129, 108 | 0 | block17_9_ac[0][0] |
| | | | block17_10_conv[0][0] |
| b̅l̅o̅c̅k̅1̅7̅_̅1̅0̅_ac (Activation) | (None, 129, 129, 108 | 0 | block17_10[0][0] |
| c̅o̅n̅v̅2̅d̅_̅1̅1̅7 (Conv2D) | (None, 129, 129, 128 | 139264 | block17_10_ac[0][0] |
| b̅a̅t̅c̅h̅_̅n̅o̅r̅malization_117 (BatchN | (None, 129, 129, 128 | 384 | conv2d_117[0][0] |
| a̅c̅t̅i̅v̅a̅t̅i̅o̅n_117 (Activation) | (None, 129, 129, 128 | 0 | |
| batch_normalization_117[0][0] | | | |
| c̅o̅n̅v̅2̅d̅_̅1̅1̅8 (Conv2D) | (None, 129, 129, 160 | 143360 | activation_117[0][0] |
| b̅a̅t̅c̅h̅_̅n̅o̅r̅malization_118 (BatchN | (None, 129, 129, 160 | 480 | conv2d_118[0][0] |
| a̅c̅t̅i̅v̅a̅t̅i̅o̅n_118 (Activation) | (None, 129, 129, 160 | 0 | |
| batch_normalization_118[0][0] | | | |
| c̅o̅n̅v̅2̅d̅_̅1̅1̅6 (Conv2D) | (None, 129, 129, 192 | 208896 | block17_10_ac[0][0] |
| c̅o̅n̅v̅2̅d̅_̅1̅1̅9 (Conv2D) | (None, 129, 129, 192 | 215040 | activation_118[0][0] |
| b̅a̅t̅c̅h̅_̅n̅o̅r̅malization_116 (BatchN | (None, 129, 129, 192 | 576 | conv2d_116[0][0] |
| b̅a̅t̅c̅h̅_̅n̅o̅r̅malization_119 (BatchN | (None, 129, 129, 192 | 576 | conv2d_119[0][0] |
| a̅c̅t̅i̅v̅a̅t̅i̅o̅n_116 (Activation) | (None, 129, 129, 192 | 0 | |
| batch_normalization_116[0][0] | | | |
| a̅c̅t̅i̅v̅a̅t̅i̅o̅n_119 (Activation) | (None, 129, 129, 192 | 0 | |
| batch_normalization_119[0][0] | | | |
| b̅l̅o̅c̅k̅1̅7̅_̅1̅1̅_mixed (Concatenate) | (None, 129, 129, 384 | 0 | activation_116[0][0] |
| | | | activation_119[0][0] |
| b̅l̅o̅c̅k̅1̅7̅_̅1̅1̅_conv (Conv2D) | (None, 129, 129, 108 | 418880 | block17_11_mixed[0][0] |
| b̅l̅o̅c̅k̅1̅7̅_̅1̅1̅ (Lambda) | (None, 129, 129, 108 | 0 | block17_10_ac[0][0] |
| | | | block17_11_conv[0][0] |
| b̅l̅o̅c̅k̅1̅7̅_̅1̅1̅_ac (Activation) | (None, 129, 129, 108 | 0 | block17_11[0][0] |
| c̅o̅n̅v̅2̅d̅_̅1̅2̅1 (Conv2D) | (None, 129, 129, 128 | 139264 | block17_11_ac[0][0] |
| b̅a̅t̅c̅h̅_̅n̅o̅r̅malization_121 (BatchN | (None, 129, 129, 128 | 384 | conv2d_121[0][0] |
| a̅c̅t̅i̅v̅a̅t̅i̅o̅n_121 (Activation) | (None, 129, 129, 128 | 0 | |
| batch_normalization_121[0][0] | | | |
| c̅o̅n̅v̅2̅d̅_̅1̅2̅2 (Conv2D) | (None, 129, 129, 160 | 143360 | activation_121[0][0] |
| b̅a̅t̅c̅h̅_̅n̅o̅r̅malization_122 (BatchN | (None, 129, 129, 160 | 480 | conv2d_122[0][0] |
| a̅c̅t̅i̅v̅a̅t̅i̅o̅n_122 (Activation) | (None, 129, 129, 160 | 0 | |
| batch_normalization_122[0][0] | | | |
| c̅o̅n̅v̅2̅d̅_̅1̅2̅0 (Conv2D) | (None, 129, 129, 192 | 208896 | block17_11_ac[0][0] |
| c̅o̅n̅v̅2̅d̅_̅1̅2̅3 (Conv2D) | (None, 129, 129, 192 | 215040 | activation_122[0][0] |
| b̅a̅t̅c̅h̅_̅n̅o̅r̅malization_120 (BatchN | (None, 129, 129, 192 | 576 | conv2d_120[0][0] |
| b̅a̅t̅c̅h̅_̅n̅o̅r̅malization_123 (BatchN | (None, 129, 129, 192 | 576 | conv2d_123[0][0] |
| a̅c̅t̅i̅v̅a̅t̅i̅o̅n_120 (Activation) | (None, 129, 129, 192 | 0 | |
| batch_normalization_120[0][0] | | | |
| a̅c̅t̅i̅v̅a̅t̅i̅o̅n_123 (Activation) | (None, 129, 129, 192 | 0 | |
| batch_normalization_123[0][0] | | | |
| b̅l̅o̅c̅k̅1̅7̅_̅1̅2̅_mixed (Concatenate) | (None, 129, 129, 384 | 0 | activation_120[0][0] |
| | | | activation_123[0][0] |
| b̅l̅o̅c̅k̅1̅7̅_̅1̅2̅_conv (Conv2D) | (None, 129, 129, 108 | 418880 | block17_12_mixed[0][0] |
| b̅l̅o̅c̅k̅1̅7̅_̅1̅2̅ (Lambda) | (None, 129, 129, 108 | 0 | block17_11_ac[0][0] |
| | | | block17_12_conv[0][0] |
| b̅l̅o̅c̅k̅1̅7̅_̅1̅2̅_ac (Activation) | (None, 129, 129, 108 | 0 | block17_12[0][0] |
| c̅o̅n̅v̅2̅d̅_̅1̅2̅5 (Conv2D) | (None, 129, 129, 128 | 139264 | block17_12_ac[0][0] |
| b̅a̅t̅c̅h̅_̅n̅o̅r̅malization_125 (BatchN | (None, 129, 129, 128 | 384 | conv2d_125[0][0] |
| a̅c̅t̅i̅v̅a̅t̅i̅o̅n_125 (Activation) | (None, 129, 129, 128 | 0 | |
| batch_normalization_125[0][0] | | | |
| c̅o̅n̅v̅2̅d̅_̅1̅2̅6 (Conv2D) | (None, 129, 129, 160 | 143360 | activation_125[0][0] |
| b̅a̅t̅c̅h̅_̅n̅o̅r̅malization_126 (BatchN | (None, 129, 129, 160 | 480 | conv2d_126[0][0] |
| a̅c̅t̅i̅v̅a̅t̅i̅o̅n_126 (Activation) | (None, 129, 129, 160 | 0 | |
| batch_normalization_126[0][0] | | | |
| c̅o̅n̅v̅2̅d̅_̅1̅2̅4 (Conv2D) | (None, 129, 129, 192 | 208896 | block17_12_ac[0][0] |
| c̅o̅n̅v̅2̅d̅_̅1̅2̅7 (Conv2D) | (None, 129, 129, 192 | 215040 | activation_126[0][0] |
| b̅a̅t̅c̅h̅_̅n̅o̅r̅malization_124 (BatchN | (None, 129, 129, 192 | 576 | conv2d_124[0][0] |
| b̅a̅t̅c̅h̅_̅n̅o̅r̅malization_127 (BatchN | (None, 129, 129, 192 | 576 | conv2d_127[0][0] |
| a̅c̅t̅i̅v̅a̅t̅i̅o̅n_124 (Activation) | (None, 129, 129, 192 | 0 | |
| batch_normalization_124[0][0] | | | |
| a̅c̅t̅i̅v̅a̅t̅i̅o̅n_127 (Activation) | (None, 129, 129, 192 | 0 | |
| batch_normalization_127[0][0] | | | |
| b̅l̅o̅c̅k̅1̅7̅_̅1̅3̅_mixed (Concatenate) | (None, 129, 129, 384 | 0 | activation_124[0][0] |
| | | | activation_127[0][0] |
| b̅l̅o̅c̅k̅1̅7̅_̅1̅3̅_conv (Conv2D) | (None, 129, 129, 108 | 418880 | block17_13_mixed[0][0] |
| b̅l̅o̅c̅k̅1̅7̅_̅1̅3̅ (Lambda) | (None, 129, 129, 108 | 0 | block17_12_ac[0][0] |
| | | | block17_13_conv[0][0] |
| b̅l̅o̅c̅k̅1̅7̅_̅1̅3̅_ac (Activation) | (None, 129, 129, 108 | 0 | block17_13[0][0] |
| c̅o̅n̅v̅2̅d̅_̅1̅2̅9 (Conv2D) | (None, 129, 129, 128 | 139264 | block17_13_ac[0][0] |
| b̅a̅t̅c̅h̅_̅n̅o̅r̅malization_129 (BatchN | (None, 129, 129, 128 | 384 | conv2d_129[0][0] |
| a̅c̅t̅i̅v̅a̅t̅i̅o̅n_129 (Activation) | (None, 129, 129, 128 | 0 | |
| batch_normalization_129[0][0] | | | |
| c̅o̅n̅v̅2̅d̅_̅1̅3̅0 (Conv2D) | (None, 129, 129, 160 | 143360 | activation_129[0][0] |
| b̅a̅t̅c̅h̅_̅n̅o̅r̅malization_130 (BatchN | (None, 129, 129, 160 | 480 | conv2d_130[0][0] |
| a̅c̅t̅i̅v̅a̅t̅i̅o̅n_130 (Activation) | (None, 129, 129, 160 | 0 | |
| batch_normalization_130[0][0] | | | |
| c̅o̅n̅v̅2̅d̅_̅1̅2̅8 (Conv2D) | (None, 129, 129, 192 | 208896 | block17_13_ac[0][0] |
| c̅o̅n̅v̅2̅d̅_̅1̅3̅1 (Conv2D) | (None, 129, 129, 192 | 215040 | activation_130[0][0] |
| b̅a̅t̅c̅h̅_̅n̅o̅r̅malization_128 (BatchN | (None, 129, 129, 192 | 576 | conv2d_128[0][0] |
| b̅a̅t̅c̅h̅_̅n̅o̅r̅malization_131 (BatchN | (None, 129, 129, 192 | 576 | conv2d_131[0][0] |
| a̅c̅t̅i̅v̅a̅t̅i̅o̅n_128 (Activation) | (None, 129, 129, 192 | 0 | |
| batch_normalization_128[0][0] | | | |
| a̅c̅t̅i̅v̅a̅t̅i̅o̅n_131 (Activation) | (None, 129, 129, 192 | 0 | |
| b̅a̅t̅c̅h̅_̅n̅o̅r̅malization_131[0][0] | | | |
| b̅l̅o̅c̅k̅1̅7̅_̅1̅4̅_mixed (Concatenate) | (None, 129, 129, 384 | 0 | activation_128[0][0] |
| | | | activation_131[0][0] |
| b̅l̅o̅c̅k̅1̅7̅_̅1̅4̅_conv (Conv2D) | (None, 129, 129, 108 | 418880 | block17_14_mixed[0][0] |
| b̅l̅o̅c̅k̅1̅7̅_̅1̅4̅ (Lambda) | (None, 129, 129, 108 | 0 | block17_13_ac[0][0] |
| | | | block17_14_conv[0][0] |
| b̅l̅o̅c̅k̅1̅7̅_̅1̅4̅_ac (Activation) | (None, 129, 129, 108 | 0 | block17_14[0][0] |
| c̅o̅n̅v̅2̅d̅_̅1̅3̅3 (Conv2D) | (None, 129, 129, 128 | 139264 | block17_14_ac[0][0] |
| b̅a̅t̅c̅h̅_̅n̅o̅r̅malization_133 (BatchN | (None, 129, 129, 128 | 384 | conv2d_133[0][0] |
| a̅c̅t̅i̅v̅a̅t̅i̅o̅n_133 (Activation) | (None, 129, 129, 128 | 0 | |
| batch_normalization_133[0][0] | | | |
| c̅o̅n̅v̅2̅d̅_̅1̅3̅4 (Conv2D) | (None, 129, 129, 160 | 143360 | activation_133[0][0] |
| b̅a̅t̅c̅h̅_̅n̅o̅r̅malization_134 (BatchN | (None, 129, 129, 160 | 480 | conv2d_134[0][0] |
| a̅c̅t̅i̅v̅a̅t̅i̅o̅n_134 (Activation) | (None, 129, 129, 160 | 0 | |
| batch_normalization_134[0][0] | | | |
| c̅o̅n̅v̅2̅d̅_̅1̅3̅2 (Conv2D) | (None, 129, 129, 192 | 208896 | block17_14_ac[0][0] |
| c̅o̅n̅v̅2̅d̅_̅1̅3̅5 (Conv2D) | (None, 129, 129, 192 | 215040 | activation_134[0][0] |
| b̅a̅t̅c̅h̅_̅n̅o̅r̅malization_132 (BatchN | (None, 129, 129, 192 | 576 | conv2d_132[0][0] |
| b̅a̅t̅c̅h̅_̅n̅o̅r̅malization_135 (BatchN | (None, 129, 129, 192 | 576 | conv2d_135[0][0] |
| a̅c̅t̅i̅v̅a̅t̅i̅o̅n_132 (Activation) | (None, 129, 129, 192 | 0 | |
| batch_normalization_132[0][0] | | | |
| a̅c̅t̅i̅v̅a̅t̅i̅o̅n_135 (Activation) | (None, 129, 129, 192 | 0 | |
| batch_normalization_135[0][0] | | | |
| b̅l̅o̅c̅k̅1̅7̅_̅1̅5̅_mixed (Concatenate) | (None, 129, 129, 384 | 0 | activation_132[0][0] |
| | | | activation_135[0][0] |
| b̅l̅o̅c̅k̅1̅7̅_̅1̅5̅_conv (Conv2D) | (None, 129, 129, 108 | 418880 | block17_15_mixed[0][0] |
| b̅l̅o̅c̅k̅1̅7̅_̅1̅5̅ (Lambda) | (None, 129, 129, 108 | 0 | block17_14_ac[0][0] |
| | | | block17_15_conv[0][0] |
| b̅l̅o̅c̅k̅1̅7̅_̅1̅5̅_ac (Activation) | (None, 129, 129, 108 | 0 | block17_15[0][0] |
| c̅o̅n̅v̅2̅d̅_̅1̅3̅7 (Conv2D) | (None, 129, 129, 128 | 139264 | block17_15_ac[0][0] |
| b̅a̅t̅c̅h̅_̅n̅o̅r̅malization_137 (BatchN | (None, 129, 129, 128 | 384 | conv2d_137[0][0] |
| a̅c̅t̅i̅v̅a̅t̅i̅o̅n_137 (Activation) | (None, 129, 129, 128 | 0 | |
| batch_normalization_137[0][0] | | | |
| c̅o̅n̅v̅2̅d̅_̅1̅3̅8 (Conv2D) | (None, 129, 129, 160 | 143360 | activation_137[0][0] |
| b̅a̅t̅c̅h̅_̅n̅o̅r̅malization_138 (BatchN | (None, 129, 129, 160 | 480 | conv2d_138[0][0] |
| a̅c̅t̅i̅v̅a̅t̅i̅o̅n_138 (Activation) | (None, 129, 129, 160 | 0 | |
| batch_normalization_138[0][0] | | | |
| c̅o̅n̅v̅2̅d̅_̅1̅3̅6 (Conv2D) | (None, 129, 129, 192 | 208896 | block17_15_ac[0][0] |
| c̅o̅n̅v̅2̅d̅_̅1̅3̅9 (Conv2D) | (None, 129, 129, 192 | 215040 | activation_138[0][0] |
| b̅a̅t̅c̅h̅_̅n̅o̅r̅malization_136 (BatchN | (None, 129, 129, 192 | 576 | conv2d_136[0][0] |
| b̅a̅t̅c̅h̅_̅n̅o̅r̅malization_139 (BatchN | (None, 129, 129, 192 | 576 | conv2d_139[0][0] |
| a̅c̅t̅i̅v̅a̅t̅i̅o̅n_136 (Activation) | (None, 129, 129, 192 | 0 | |
| batch_normalization_136[0][0] | | | |
| a̅c̅t̅i̅v̅a̅t̅i̅o̅n_139 (Activation) batch_normalization_139[0][0] | (None, 129, 129, 192 | 0 | |
| b̅l̅o̅c̅k̅1̅7̅_̅1̅6̅_mixed (Concatenate) | (None, 129, 129, 384 | 0 | activation_136[0][0] |
| | | | activation_139[0][0] |
| b̅l̅o̅c̅k̅1̅7̅_̅1̅6̅_conv (Conv2D) | (None, 129, 129, 108 | 418880 | block17_16_mixed[0][0] |
| b̅l̅o̅c̅k̅1̅7̅_̅1̅6̅ (Lambda) | (None, 129, 129, 108 | 0 | block17_15_ac[0][0] |
| | | | block17_16_conv[0][0] |
| b̅l̅o̅c̅k̅1̅7̅_̅1̅6̅_ac (Activation) | (None, 129, 129, 108 | 0 | block17_16[0][0] |
| c̅o̅n̅v̅2̅d̅_̅1̅4̅1 (Conv2D) | (None, 129, 129, 128 | 139264 | block17_16_ac[0][0] |
| b̅a̅t̅c̅h̅_̅n̅o̅r̅malization_141 (BatchN | (None, 129, 129, 128 | 384 | conv2d_141[0][0] |
| a̅c̅t̅i̅v̅a̅t̅i̅o̅n_141 (Activation) | (None, 129, 129, 128 | 0 | |
| batch_normalization_141[0][0] | | | |
| c̅o̅n̅v̅2̅d̅_̅1̅4̅2 (Conv2D) | (None, 129, 129, 160 | 143360 | activation_141[0][0] |
| b̅a̅t̅c̅h̅_̅n̅o̅r̅malization_142 (BatchN | (None, 129, 129, 160 | 480 | conv2d_142[0][0] |
| a̅c̅t̅i̅v̅a̅t̅i̅o̅n_142 (Activation) | (None, 129, 129, 160 | 0 | |
| batch_normalization_142[0][0] | | | |
| c̅o̅n̅v̅2̅d̅_̅1̅4̅0 (Conv2D) | (None, 129, 129, 192 | 208896 | block17_16_ac[0][0] |
| c̅o̅n̅v̅2̅d̅_̅1̅4̅3 (Conv2D) | (None, 129, 129, 192 | 215040 | activation_142[0][0] |
| b̅a̅t̅c̅h̅_̅n̅o̅r̅malization_140 (BatchN | (None, 129, 129, 192 | 576 | conv2d_140[0][0] |
| b̅a̅t̅c̅h̅_̅n̅o̅r̅malization_143 (BatchN | (None, 129, 129, 192 | 576 | conv2d_143[0][0] |
| a̅c̅t̅i̅v̅a̅t̅i̅o̅n_140 (Activation) | (None, 129, 129, 192 | 0 | |
| batch_normalization_140[0][0] | | | |
| a̅c̅t̅i̅v̅a̅t̅i̅o̅n_143 (Activation) | (None, 129, 129, 192 | 0 | |
| batch_normalization_143[0][0] | | | |
| b̅l̅o̅c̅k̅1̅7̅_̅1̅7̅_mixed (Concatenate) | (None, 129, 129, 384 | 0 | activation_140[0][0] |
| | | | activation_143[0][0] |
| b̅l̅o̅c̅k̅1̅7̅_̅1̅7̅_conv (Conv2D) | (None, 129, 129, 108 | 418880 | block17_17_mixed[0][0] |
| b̅l̅o̅c̅k̅1̅7̅_̅1̅7̅ (Lambda) | (None, 129, 129, 108 | 0 | block17_16_ac[0][0] |
| | | | block17_17_conv[0][0] |
| b̅l̅o̅c̅k̅1̅7̅_̅1̅7̅_ac (Activation) | (None, 129, 129, 108 | 0 | block17_17[0][0] |
| c̅o̅n̅v̅2̅d̅_̅1̅4̅5 (Conv2D) | (None, 129, 129, 128 | 139264 | block17_17_ac[0][0] |
| b̅a̅t̅c̅h̅_̅n̅o̅r̅malization_145 (BatchN | (None, 129, 129, 128 | 384 | conv2d_145[0][0] |
| a̅c̅t̅i̅v̅a̅t̅i̅o̅n_145 (Activation) | (None, 129, 129, 128 | 0 | |
| batch_normalization_145[0][0] | | | |
| c̅o̅n̅v̅2̅d̅_̅1̅4̅6 (Conv2D) | (None, 129, 129, 160 | 143360 | activation_145[0][0] |
| b̅a̅t̅c̅h̅_̅n̅o̅r̅malization_146 (BatchN | (None, 129, 129, 160 | 480 | conv2d_146[0][0] |
| a̅c̅t̅i̅v̅a̅t̅i̅o̅n_146 (Activation) | (None, 129, 129, 160 | 0 | |
| batch_normalization_146[0][0] | | | |
| c̅o̅n̅v̅2̅d̅_̅1̅4̅4 (Conv2D) | (None, 129, 129, 192 | 208896 | block17_17_ac[0][0] |
| c̅o̅n̅v̅2̅d̅_̅1̅4̅7 (Conv2D) | (None, 129, 129, 192 | 215040 | activation_146[0][0] |
| b̅a̅t̅c̅h̅_̅n̅o̅r̅malization_144 (BatchN | (None, 129, 129, 192 | 576 | conv2d_144[0][0] |
| b̅a̅t̅c̅h̅_̅n̅o̅r̅malization_147 (BatchN | (None, 129, 129, 192 | 576 | conv2d_147[0][0] |
| a̅c̅t̅i̅v̅a̅t̅i̅o̅n_144 (Activation) | (None, 129, 129, 192 | 0 | |
| batch_normalization_144[0][0] | | | |
| a̅c̅t̅i̅v̅a̅t̅i̅o̅n_147 (Activation) | (None, 129, 129, 192 | 0 | |
| batch_normalization_147[0][0] | | | |
| b̅l̅o̅c̅k̅1̅7̅_̅1̅8̅_mixed (Concatenate) | (None, 129, 129, 384 | 0 | activation_144[0][0] |
| | | | activation_147[0][0] |
| b̅l̅o̅c̅k̅1̅7̅_̅1̅8̅_conv (Conv2D) | (None, 129, 129, 108 | 418880 | block17_18_mixed[0][0] |
| b̅l̅o̅c̅k̅1̅7̅_̅1̅8̅ (Lambda) | (None, 129, 129, 108 | 0 | block17_17_ac[0][0] |
| | | | block17_18_conv[0][0] |
| b̅l̅o̅c̅k̅1̅7̅_̅1̅8̅_ac (Activation) | (None, 129, 129, 108 | 0 | block17_18[0][0] |
| c̅o̅n̅v̅2̅d̅_̅1̅4̅9 (Conv2D) | (None, 129, 129, 128 | 139264 | block17_18_ac[0][0] |
| b̅a̅t̅c̅h̅_̅n̅o̅r̅malization_149 (BatchN | (None, 129, 129, 128 | 384 | conv2d_149[0][0] |
| a̅c̅t̅i̅v̅a̅t̅i̅o̅n_149 (Activation) | (None, 129, 129, 128 | 0 | |
| batch_normalization_149[0][0] | | | |
| c̅o̅n̅v̅2̅d̅_̅1̅5̅0 (Conv2D) | (None, 129, 129, 160 | 143360 | activation_149[0][0] |
| b̅a̅t̅c̅h̅_̅n̅o̅r̅malization_150 (BatchN | (None, 129, 129, 160 | 480 | conv2d_50[0][0] |
| a̅c̅t̅i̅v̅a̅t̅i̅o̅n_150 (Activation) | (None, 129, 129, 160 | 0 | |
| batch_normalization_150[0] [0] | | | |
| c̅o̅n̅v̅2̅d̅_̅1̅4̅8 (Conv2D) | (None, 129, 129, 192 | 208896 | block17_18_ac[0][0] |
| c̅o̅n̅v̅2̅d̅_̅1̅5̅1 (Conv2D) | (None, 129, 129, 192 | 215040 | activation_150[0][0] |
| b̅a̅t̅c̅h̅_̅n̅o̅r̅malization_148 (BatchN | (None, 129, 129, 192 | 576 | conv2d_148[0][0] |
| b̅a̅t̅c̅h̅_̅n̅o̅r̅malization_151 (BatchN | (None, 129, 129, 192 | 576 | conv2d_151[0][0] |
| a̅c̅t̅i̅v̅a̅t̅i̅o̅n_148 (Activation) | (None, 129, 129, 192 | 0 | |
| batch_normalization_148[0][0] | | | |
| a̅c̅t̅i̅v̅a̅t̅i̅o̅n_151 (Activation) | (None, 129, 129, 192 | 0 | |
| batch_normalization_151[0][0] | | | |
| b̅l̅o̅c̅k̅1̅7̅_̅1̅9̅_mixed (Concatenate) | (None, 129, 129, 384 | 0 | activation_148[0][0] |
| | | | activation_151[0][0] |
| b̅l̅o̅c̅k̅1̅7̅_̅1̅9̅_conv (Conv2D) | (None, 129, 129, 108 | 418880 | block17_19_mixed[0][0] |
| b̅l̅o̅c̅k̅1̅7̅_̅1̅9̅ (Lambda) | (None, 129, 129, 108 | 0 | block17_18_ac[0][0] |
| | | | block17_19_conv[0][0] |
| b̅l̅o̅c̅k̅1̅7̅_̅1̅9̅_ac (Activation) | (None, 129, 129, 108 | 0 | block17_19[0][0] |
| c̅o̅n̅v̅2̅d̅_̅1̅5̅3 (Conv2D) | (None, 129, 129, 128 | 139264 | block17_19_ac[0][0] |
| b̅a̅t̅c̅h̅_̅n̅o̅r̅malization_153 (BatchN | (None, 129, 129, 128 | 384 | conv2d_153[0][0] |
| a̅c̅t̅i̅v̅a̅t̅i̅o̅n_153 (Activation) | (None, 129, 129, 128 | 0 | |
| batch_normalization_153[0][0] | | | |
| c̅o̅n̅v̅2̅d̅_̅1̅5̅4 (Conv2D) | (None, 129, 129, 160 | 143360 | activation_153[0][0] |
| b̅a̅t̅c̅h̅_̅n̅o̅r̅malization_154 (BatchN | (None, 129, 129, 160 | 480 | conv2d_154[0][0] |
| a̅c̅t̅i̅v̅a̅t̅i̅o̅n_154 (Activation) | (None, 129, 129, 160 | 0 | |
| batch_normalization_154[0][0] | | | |
| c̅o̅n̅v̅2̅d̅_̅1̅5̅2 (Conv2D) | (None, 129, 129, 192 | 208896 | block17_19_ac[0][0] |
| c̅o̅n̅v̅2̅d̅_̅1̅5̅5 (Conv2D) | (None, 129, 129, 192 | 215040 | activation_154[0][0] |
| b̅a̅t̅c̅h̅_̅n̅o̅r̅malization_152 (BatchN | (None, 129, 129, 192 | 576 | conv2d_152[0][0] |
| b̅a̅t̅c̅h̅_̅n̅o̅r̅malization_155 (BatchN | (None, 129, 129, 192 | 576 | conv2d_155[0][0] |
| a̅c̅t̅i̅v̅a̅t̅i̅o̅n_152 (Activation) | (None, 129, 129, 192 | 0 | |
| batch_normalization_152[0][0] | | | |
| a̅c̅t̅i̅v̅a̅t̅i̅o̅n_155 (Activation) | (None, 129, 129, 192 | 0 | |
| batch_normalization_155[0][0] | | | |
| b̅l̅o̅c̅k̅1̅7̅_̅2̅0̅_mixed (Concatenate) | (None, 129, 129, 384 | 0 | activation_152[0][0] |
| | | | activation_155[0][0] |
| b̅l̅o̅c̅k̅1̅7̅_̅2̅0̅_conv (Conv2D) | (None, 129, 129, 108 | 418880 | block17_20_mixed[0][0] |
| b̅l̅o̅c̅k̅1̅7̅_̅2̅0̅ (Lambda) | (None, 129, 129, 108 | 0 | block17_19_ac[0][0] |
| | | | block17_20_conv[0][0] |
| b̅l̅o̅c̅k̅1̅7̅_̅2̅0̅_ac (Activation) | (None, 129, 129, 108 | 0 | block17_20[0][0] |
| c̅o̅n̅v̅2̅d̅_̅1̅6̅0 (Conv2D) | (None, 129, 129, 256 | 278528 | block17_20_ac[0][0] |
| b̅a̅t̅c̅h̅_̅n̅o̅r̅malization_160 (BatchN | (None, 129, 129, 256 | 768 | conv2d_160[0][0] |
| a̅c̅t̅i̅v̅a̅t̅i̅o̅n_160 (Activation) | (None, 129, 129, 256 | 0 | |
| batch_normalization_160[0][0] | | | |
| c̅o̅n̅v̅2̅d̅_̅1̅5̅6 (Conv2D) | (None, 129, 129, 256 | 278528 | block17_20_ac[0][0] |
| c̅o̅n̅v̅2̅d̅_̅1̅5̅8 (Conv2D) | (None, 129, 129, 256 | 278528 | block17_20_ac[0][0] |
| c̅o̅n̅v̅2̅d̅_̅1̅6̅1 (Conv2D) | (None, 129, 129, 288 | 663552 | activation_160[0][0] |
| b̅a̅t̅c̅h̅_̅n̅o̅r̅malization_156 (BatchN | (None, 129, 129, 256 | 768 | conv2d_156[0][0] |
| b̅a̅t̅c̅h̅_̅n̅o̅r̅malization_158 (BatchN | (None, 129, 129, 256 | 768 | conv2d_158[0][0] |
| b̅a̅t̅c̅h̅_̅n̅o̅r̅malization_161 (BatchN | (None, 129, 129, 288 | 864 | conv2d_161[0][0] |
| a̅c̅t̅i̅v̅a̅t̅i̅o̅n̅_̅1̅5̅6 (Activation) | (None, 129, 129, 256 | 0 | |
| batch_normalization_156[0][0] | | | |
| a̅c̅t̅i̅v̅a̅t̅i̅o̅n_158 (Activation) | (None, 129, 129, 256 | 0 | |
| batch_normalization_158[0][0] | | | |
| a̅c̅t̅i̅v̅a̅t̅i̅o̅n_161 (Activation) | (None, 129, 129, 288 | 0 | |
| batch_normalization_161[0][0] | | | |
| c̅o̅n̅v̅2̅d̅_̅1̅5̅7 (Conv2D) | (None, 64, 64, 384) | 884736 | activation_156[0][0] |
| c̅o̅n̅v̅2̅d̅_̅1̅5̅9 (Conv2D) | (None, 64, 64, 288) | 663552 | activation_158[0][0] |
| c̅o̅n̅v̅2̅d̅_̅1̅6̅2 (Conv2D) | (None, 64, 64, 320) | 829440 | activation_161[0][0] |
| b̅a̅t̅c̅h̅_̅n̅o̅r̅malization_157 (BatchN | (None, 64, 64, 384) | 1152 | conv2d_157[0][0] |
| b̅a̅t̅c̅h̅_̅n̅o̅r̅malization_159 (BatchN | (None, 64, 64, 288) | 864 | conv2d_159[0][0] |
| b̅a̅t̅c̅h̅_̅n̅o̅r̅malization_162 (BatchN | (None, 64, 64, 320) | 960 | conv2d_162[0][0] |
| a̅c̅t̅i̅v̅a̅t̅i̅o̅n_157 (Activation) | (None, 64, 64, 384) | 0 | |
| batch_normalization_157[0][0] | | | |
| a̅c̅t̅i̅v̅a̅t̅i̅o̅n_159 (Activation) | (None, 64, 64, 288) | 0 | |
| batch_normalization_159[0][0] | | | |
| a̅c̅t̅i̅v̅a̅t̅i̅o̅n_162 (Activation) | (None, 64, 64, 320) | 0 | |
| batch_normalization_162[0][0] | | | |
| m̅a̅x̅_̅p̅o̅o̅l̅i̅ng2d_3 (MaxPooling2D) | (None, 64, 64, 1088) | 0 | block17_20_ac[0][0] |
| m̅i̅x̅e̅d̅_̅7̅a̅ (Concatenate) | (None, 64, 64, 2080) | 0 | activation_157[0][0] |
| | | | activation_159[0][0] |
| | | | activation_162[0][0] |
| | | | max_pooling2d_3[0][0] |
| c̅o̅n̅v̅2̅d̅_̅1̅6̅4 (Conv2D) | (None, 64, 64, 192) | 399360 | mixed_7a[0][0] |
| b̅a̅t̅c̅h̅_̅n̅o̅r̅malization_164 (BatchN | (None, 64, 64, 192) | 576 | conv2d_164[0][0] |
| a̅c̅t̅i̅v̅a̅t̅i̅o̅n_164 (Activation) | (None, 64, 64, 192) | 0 | |
| batch_normalization_164[0][0] | | | |
| c̅o̅n̅v̅2̅d̅_̅1̅6̅5 (Conv2D) | (None, 64, 64, 224) | 129024 | activation_164[0][0] |
| b̅a̅t̅c̅h̅_̅n̅o̅r̅malization_165 (BatchN | (None, 64, 64, 224) | 672 | conv2d_165[0][0] |
| a̅c̅t̅i̅v̅a̅t̅i̅o̅n_165 (Activation) | (None, 64, 64, 224) | 0 | |
| batch_normalization_165[0][0] | | | |
| c̅o̅n̅v̅2̅d̅_̅1̅6̅3 (Conv2D) | (None, 64, 64, 192) | 399360 | mixed_7a[0][0] |
| c̅o̅n̅v̅2̅d̅_̅1̅6̅6 (Conv2D) | (None, 64, 64, 256) | 172032 | activation_165[0][0] |
| b̅a̅t̅c̅h̅_̅n̅o̅r̅malization_163 (BatchN | (None, 64, 64, 192) | 576 | conv2d_163[0][0] |
| b̅a̅t̅c̅h̅_̅n̅o̅r̅malization_166 (BatchN | (None, 64, 64, 256) | 768 | conv2d_166[0][0] |
| a̅c̅t̅i̅v̅a̅t̅i̅o̅n_163 (Activation) | (None, 64, 64, 192) | 0 | |
| batch_normalization_163[0][0] | | | |
| a̅c̅t̅i̅v̅a̅t̅i̅o̅n_166 (Activation) | (None, 64, 64, 256) | 0 | |
| batch_normalization_166[0][0] | | | |
| b̅l̅o̅c̅k̅8̅_̅1̅_mixed (Concatenate) | (None, 64, 64, 448) | 0 | activation_163[0][0] |
| | | | activation_166[0][0] |
| b̅l̅o̅c̅k̅8̅_̅1̅_conv (Conv2D) | (None, 64, 64, 2080) | 933920 | block8_1_mixed[0][0] |
| b̅l̅o̅c̅k̅8̅_̅1̅ (Lambda) | (None, 64, 64, 2080) | 0 | mixed_7a[0][0] block8_1_conv[0][0] |
| b̅l̅o̅c̅k̅8̅_̅1̅_ac (Activation) | (None, 64, 64, 2080) | 0 | block8_1[0][0] |
| c̅o̅n̅v̅2̅d̅_̅1̅6̅8 (Conv2D) | (None, 64, 64, 192) | 399360 | block8_1_ac[0][0] |
| b̅a̅t̅c̅h̅_̅n̅o̅r̅malization_168 (BatchN | (None, 64, 64, 192) | 576 | conv2d_168[0][0] |
| a̅c̅t̅i̅v̅a̅t̅i̅o̅n_168 (Activation) | (None, 64, 64, 192) | 0 | |
| batch_normalization_168[0][0] | | | |
| c̅o̅n̅v̅2̅d̅_̅1̅6̅9 (Conv2D) | (None, 64, 64, 224) | 129024 | activation_168[0][0] |
| b̅a̅t̅c̅h̅_̅n̅o̅r̅malization_169 (BatchN | (None, 64, 64, 224) | 672 | conv2d_169[0][0] |
| a̅c̅t̅i̅v̅a̅t̅i̅o̅n_169 (Activation) | (None, 64, 64, 224) | 0 | |
| batch_normalization_169[0][0] | | | |
| c̅o̅n̅v̅2̅d̅_̅1̅6̅7 (Conv2D) | (None, 64, 64, 192) | 399360 | block8_1_ac[0][0] |
| c̅o̅n̅v̅2̅d̅_̅1̅7̅0 (Conv2D) | (None, 64, 64, 256) | 172032 | activation_169[0][0] |
| b̅a̅t̅c̅h̅_̅n̅o̅r̅malization_167 (BatchN | (None, 64, 64, 192) | 576 | conv2d_167[0][0] |
| b̅a̅t̅c̅h̅_̅n̅o̅r̅malization_170 (BatchN | (None, 64, 64, 256) | 768 | conv2d_170[0][0] |
| a̅c̅t̅i̅v̅a̅t̅i̅o̅n_167 (Activation) | (None, 64, 64, 192) | 0 | |
| batch_normalization_167[0][0] | | | |
| a̅c̅t̅i̅v̅a̅t̅i̅o̅n_170 (Activation) | (None, 64, 64, 256) | 0 | |
| batch_normalization_170[0][0] | | | |
| b̅l̅o̅c̅k̅8̅_̅2̅_mixed (Concatenate) | (None, 64, 64, 448) | 0 | activation_167[0][0] |
| | | | activation_170[0][0] |
| b̅l̅o̅c̅k̅8̅_̅2̅_conv (Conv2D) | (None, 64, 64, 2080) | 933920 | block8_2_mixed[0][0] |
| b̅l̅o̅c̅k̅8̅_̅2̅ (Lambda) | (None, 64, 64, 2080) | 0 | block8_1_ac[0][0] |
| | | | block8_2_conv[0][0] |
| b̅l̅o̅c̅k̅8̅_̅2̅_ac (Activation) | (None, 64, 64, 2080) | 0 | block8_2[0][0] |
| c̅o̅n̅v̅2̅d̅_̅1̅7̅2 (Conv2D) | (None, 64, 64, 192) | 399360 | block8_2_ac[0][0] |
| b̅a̅t̅c̅h̅_̅n̅o̅r̅malization_172 (BatchN | (None, 64, 64, 192) | 576 | conv2d_172[0][0] |
| a̅c̅t̅i̅v̅a̅t̅i̅o̅n_172 (Activation) | (None, 64, 64, 192) | 0 | |
| batch_normalization_172[0][0] | | | |
| c̅o̅n̅v̅2̅d̅_̅1̅7̅3 (Conv2D) | (None, 64, 64, 224) | 129024 | activation_172[0][0] |
| b̅a̅t̅c̅h̅_̅n̅o̅r̅malization_173 (BatchN | (None, 64, 64, 224) | 672 | conv2d_173[0][0] |
| a̅c̅t̅i̅v̅a̅t̅i̅o̅n_173 (Activation) | (None, 64, 64, 224) | 0 | |
| batch_normalization_173[0][0] | | | |
| c̅o̅n̅v̅2̅d̅_̅1̅7̅1 (Conv2D) | (None, 64, 64, 192) | 399360 | block8_2_ac[0][0] |
| c̅o̅n̅v̅2̅d̅_̅1̅7̅4 (Conv2D) | (None, 64, 64, 256) | 172032 | activation_173[0][0] |
| b̅a̅t̅c̅h̅_̅n̅o̅r̅malization_171 (BatchN | (None, 64, 64, 192) | 576 | conv2d_171[0][0] |
| b̅a̅t̅c̅h̅_̅n̅o̅r̅malization_174 (BatchN | (None, 64, 64, 256) | 768 | conv2d_174[0][0] |
| a̅c̅t̅i̅v̅a̅t̅i̅o̅n_171 (Activation) | (None, 64, 64, 192) | 0 | |
| batch_normalization_171[0][0] | | | |
| a̅c̅t̅i̅v̅a̅t̅i̅o̅n_174 (Activation) | (None, 64, 64, 256) | 0 | |
| batch_normalization_174[0][0] | | | |
| b̅l̅o̅c̅k̅8̅_̅3̅_mixed (Concatenate) | (None, 64, 64, 448) | 0 | activation_171[0][0] |
| | | | activation_174[0][0] |
| b̅l̅o̅c̅k̅8̅_̅3̅_conv (Conv2D) | (None, 64, 64, 2080) | 933920 | block8_3_mixed[0][0] |
| b̅l̅o̅c̅k̅8̅_̅3̅ (Lambda) | (None, 64, 64, 2080) | 0 | block8_2_ac[0][0] |
| | | | block8_3_conv[0][0] |
| b̅l̅o̅c̅k̅8̅_̅3̅_ac (Activation) | (None, 64, 64, 2080) | 0 | block8_3[0][0] |
| c̅o̅n̅v̅2̅d̅_̅1̅7̅6 (Conv2D) | (None, 64, 64, 192) | 399360 | block8_3_ac[0][0] |
| b̅a̅t̅c̅h̅_̅n̅o̅r̅malization_176 (BatchN | (None, 64, 64, 192) | 576 | conv2d_176[0][0] |
| a̅c̅t̅i̅v̅a̅t̅i̅o̅n_176 (Activation) | (None, 64, 64, 192) | 0 | |
| batch_normalization_176[0][0] | | | |
| c̅o̅n̅v̅2̅d̅_̅1̅7̅7 (Conv2D) | (None, 64, 64, 224) | 129024 | activation_176[0][0] |
| b̅a̅t̅c̅h̅_̅n̅o̅r̅malization_177 (BatchN | (None, 64, 64, 224) | 672 | conv2d_177[0][0] |
| a̅c̅t̅i̅v̅a̅t̅i̅o̅n_177 (Activation) | (None, 64, 64, 224) | 0 | |
| batch_normalization_177[0][0] | | | |
| c̅o̅n̅v̅2̅d̅_̅1̅7̅5 (Conv2D) | (None, 64, 64, 192) | 399360 | block8_3_ac[0][0] |
| c̅o̅n̅v̅2̅d̅_̅1̅7̅8 (Conv2D) | (None, 64, 64, 256) | 172032 | activation_177[0][0] |
| b̅a̅t̅c̅h̅_̅n̅o̅r̅malization_175 (BatchN | (None, 64, 64, 192) | 576 | conv2d_175[0][0] |
| b̅a̅t̅c̅h̅_̅n̅o̅r̅normalization_178 (BatchN | (None, 64, 64, 256) | 768 | conv2d_178[0][0] |
| a̅c̅t̅i̅v̅a̅t̅i̅o̅n_175 (Activation) | (None, 64, 64, 192) | 0 | |
| batch_normalization_175[0][0] | | | |
| a̅c̅t̅i̅v̅a̅t̅i̅o̅n_178 (Activation) | (None, 64, 64, 256) | 0 | |
| batch_normalization_178[0][0] | | | |
| b̅l̅o̅c̅k̅8̅_̅4̅_mixed (Concatenate) | (None, 64, 64, 448) | 0 | activation_175[0][0] |
| | | | activation_178[0][0] |
| b̅l̅o̅c̅k̅8̅_̅4̅_conv (Conv2D) | (None, 64, 64, 2080) | 933920 | block8_4_mixed[0][0] |
| b̅l̅o̅c̅k̅8̅_̅4̅ (Lambda) | (None, 64, 64, 2080) | 0 | block8_3_ac[0][0] |
| | | | block8_4_conv[0][0] |
| b̅l̅o̅c̅k̅8̅_̅4̅_ac (Activation) | (None, 64, 64, 2080) | 0 | block8_4[0][0] |
| c̅o̅n̅v̅2̅d̅_̅1̅8̅0 (Conv2D) | (None, 64, 64, 192) | 399360 | block8_4_ac[0][0] |
| b̅a̅t̅c̅h̅_̅n̅o̅r̅malization_180 (BatchN | (None, 64, 64, 192) | 576 | conv2d_180[0][0] |
| a̅c̅t̅i̅v̅a̅t̅i̅o̅n_180 (Activation) | (None, 64, 64, 192) | 0 | |
| batch_normalization_180[0][0] | | | |
| c̅o̅n̅v̅2̅d̅_̅1̅8̅1 (Conv2D) | (None, 64, 64, 224) | 129024 | activation_180[0][0] |
| b̅a̅t̅c̅h̅_̅n̅o̅r̅malization_181 (BatchN | (None, 64, 64, 224) | 672 | conv2d_181[0][0] |
| a̅c̅t̅i̅v̅a̅t̅i̅o̅n_181 (Activation) | (None, 64, 64, 224) | 0 | |
| batch_normalization_181[0][0] | | | |
| c̅o̅n̅v̅2̅d̅_̅1̅7̅9 (Conv2D) | (None, 64, 64, 192) | 399360 | block8_4_ac[0][0] |
| c̅o̅n̅v̅2̅d̅_̅1̅8̅2 (Conv2D) | (None, 64, 64, 256) | 172032 | activation_181[0][0] |
| b̅a̅t̅c̅h̅_̅n̅o̅r̅malization_179 (BatchN | (None, 64, 64, 192) | 576 | conv2d_179[0][0] |
| b̅a̅t̅c̅h̅_̅n̅o̅r̅malization_182 (BatchN | (None, 64, 64, 256) | 768 | conv2d_182[0][0] |
| a̅c̅t̅i̅v̅a̅t̅i̅o̅n_179 (Activation) | (None, 64, 64, 192) | 0 | |
| batch_normalization_179[0][0] | | | |
| a̅c̅t̅i̅v̅a̅t̅i̅o̅n_182 (Activation) | (None, 64, 64, 256) | 0 | |
| batch_normalization_182[0][0] | | | |
| b̅l̅o̅c̅k̅8̅_̅5̅_mixed (Concatenate) | (None, 64, 64, 448) | 0 | activation_179[0][0] |
| | | | activation_182[0][0] |
| b̅l̅o̅c̅k̅8̅_̅5̅_conv (Conv2D) | (None, 64, 64, 2080) | 933920 | block8_5_mixed[0][0] |
| b̅l̅o̅c̅k̅8̅_̅5̅ (Lambda) | (None, 64, 64, 2080) | 0 | block8_4_ac[0][0] |
| | | | block8_5_conv[0][0] |
| b̅l̅o̅c̅k̅8̅_̅5̅_ac (Activation) | (None, 64, 64, 2080) | 0 | block8_5[0][0] |
| c̅o̅n̅v̅2̅d̅_̅1̅8̅4 (Conv2D) | (None, 64, 64, 192) | 399360 | block8_5_ac[0][0] |
| b̅a̅t̅c̅h̅_̅n̅o̅r̅malization_184 (BatchN | (None, 64, 64, 192) | 576 | conv2d_184[0][0] |
| a̅c̅t̅i̅v̅a̅t̅i̅o̅n_184 (Activation) | (None, 64, 64, 192) | 0 | |
| batch_normalization_184[0][0] | | | |
| c̅o̅n̅v̅2̅d̅_̅1̅8̅5 (Conv2D) | (None, 64, 64, 224) | 129024 | activation_184[0][0] |
| b̅a̅t̅c̅h̅_̅n̅o̅r̅malization_185 (BatchN | (None, 64, 64, 224) | 672 | conv2d_185[0][0] |
| a̅c̅t̅i̅v̅a̅t̅i̅o̅n_185 (Activation) | (None, 64, 64, 224) | 0 | |
| batch_normalization_185[0][0] | | | |
| c̅o̅n̅v̅2̅d̅_̅1̅8̅3 (Conv2D) | (None, 64, 64, 192) | 399360 | block8_5_ac[0][0] |
| c̅o̅n̅v̅2̅d̅_̅1̅8̅6 (Conv2D) | (None, 64, 64, 256) | 172032 | activation_185[0][0] |
| b̅a̅t̅c̅h̅_̅n̅o̅r̅malization_183 (BatchN | (None, 64, 64, 192) | 576 | conv2d_183[0][0] |
| b̅a̅t̅c̅h̅_̅n̅o̅r̅malization_186 (BatchN | (None, 64, 64, 256) | 768 | conv2d_186[0][0] |
| a̅c̅t̅i̅v̅a̅t̅i̅o̅n_183 (Activation) | (None, 64, 64, 192) | 0 | |
| batch_normalization_183[0][0] | | | |
| a̅c̅t̅i̅v̅a̅t̅i̅o̅n_186 (Activation) | (None, 64, 64, 256) | 0 | |
| batch_normalization_186[0][0] | | | |
| b̅l̅o̅c̅k̅8̅_̅6̅_mixed (Concatenate) | (None, 64, 64, 448) | 0 | activation_183[0][0] |
| | | | activation_186[0][0] |
| b̅l̅o̅c̅k̅8̅_̅6̅_conv (Conv2D) | (None, 64, 64, 2080) | 933920 | block8_6_mixed[0][0] |
| b̅l̅o̅c̅k̅8̅_̅6̅ (Lambda) | (None, 64, 64, 2080) | 0 | block8_5_ac[0][0] |
| | | | block8_6_conv[0][0] |
| b̅l̅o̅c̅k̅8̅_̅6̅_ac (Activation) | (None, 64, 64, 2080) | 0 | block8_6[0][0] |
| c̅o̅n̅v̅2̅d̅_̅1̅8̅8 (Conv2D) | (None, 64, 64, 192) | 399360 | block8_6_ac[0][0] |
| b̅a̅t̅c̅h̅_̅n̅o̅r̅malization_188 (BatchN | (None, 64, 64, 192) | 576 | conv2d_188[0][0] |
| a̅c̅t̅i̅v̅a̅t̅i̅o̅n_188 (Activation) | (None, 64, 64, 192) | 0 | |
| batch_normalization_188[0][0] | | | |
| c̅o̅n̅v̅2̅d̅_̅1̅8̅9 (Conv2D) | (None, 64, 64, 224) | 129024 | activation_188[0][0] |
| b̅a̅t̅c̅h̅_̅n̅o̅r̅malization_189 (BatchN | (None, 64, 64, 224) | 672 | conv2d_189[0][0] |
| a̅c̅t̅i̅v̅a̅t̅i̅o̅n_189 (Activation) | (None, 64, 64, 224) | 0 | |
| batch_normalization_189[0][0] | | | |
| c̅o̅n̅v̅2̅d̅_̅1̅8̅7 (Conv2D) | (None, 64, 64, 192) | 399360 | block8_6_ac[0][0] |
| c̅o̅n̅v̅2̅d̅_̅1̅9̅0 (Conv2D) | (None, 64, 64, 256) | 172032 | activation_189[0][0] |
| b̅a̅t̅c̅h̅_̅n̅o̅r̅malization_187 (BatchN | (None, 64, 64, 192) | 576 | conv2d_187[0][0] |
| b̅a̅t̅c̅h̅_̅n̅o̅r̅malization_190 (BatchN | (None, 64, 64, 256) | 768 | conv2d_190[0][0] |
| a̅c̅t̅i̅v̅a̅t̅i̅o̅n_187 (Activation) | (None, 64, 64, 192) | 0 | |
| batch_normalization_187[0][0] | | | |
| a̅c̅t̅i̅v̅a̅t̅i̅o̅n_190 (Activation) | (None, 64, 64, 256) | 0 | |
| batch_normalization_190[0][0] | | | |
| b̅l̅o̅c̅k̅8̅_̅7̅_mixed (Concatenate) | (None, 64, 64, 448) | 0 | activation_187[0][0] |
| | | | activation_190[0][0] |
| b̅l̅o̅c̅k̅8̅_̅7̅_conv (Conv2D) | (None, 64, 64, 2080) | 933920 | block8_7_mixed[0][0] |
| b̅l̅o̅c̅k̅8̅_̅7̅ (Lambda) | (None, 64, 64, 2080) | 0 | block8_6_ac[0][0] |
| | | | block8_7_conv[0][0] |
| b̅l̅o̅c̅k̅8̅_̅7̅_ac (Activation) | (None, 64, 64, 2080) | 0 | block8_7[0][0] |
| c̅o̅n̅v̅2̅d̅_̅1̅9̅2 (Conv2D) | (None, 64, 64, 192) | 399360 | block8_7_ac[0][0] |
| b̅a̅t̅c̅h̅_̅n̅o̅r̅malization_192 (BatchN | (None, 64, 64, 192) | 576 | conv2d_192[0][0] |
| a̅c̅t̅i̅v̅a̅t̅i̅o̅n_192 (Activation) | (None, 64, 64, 192) | 0 | |
| batch_normalization_192[0][0] | | | |
| c̅o̅n̅v̅2̅d̅_̅1̅9̅3 (Conv2D) | (None, 64, 64, 224) | 129024 | activation_192[0][0] |
| b̅a̅t̅c̅h̅_̅n̅o̅r̅malization_193 (BatchN | (None, 64, 64, 224) | 672 | conv2d_193[0][0] |
| a̅c̅t̅i̅v̅a̅t̅i̅o̅n_193 (Activation) | (None, 64, 64, 224) | 0 | |
| batch_normalization_193[0][0] | | | |
| c̅o̅n̅v̅2̅d̅_̅1̅9̅1 (Conv2D) | (None, 64, 64, 192) | 399360 | block8_7_ac[0][0] |
| c̅o̅n̅v̅2̅d̅_̅1̅9̅4 (Conv2D) | (None, 64, 64, 256) | 172032 | activation_193[0][0] |
| b̅a̅t̅c̅h̅_̅n̅o̅r̅malization_191 (BatchN | (None, 64, 64, 192) | 576 | conv2d_191[0][0] |
| b̅a̅t̅c̅h̅_̅n̅o̅r̅malization_194 (BatchN | (None, 64, 64, 256) | 768 | conv2d_194[0][0] |
| a̅c̅t̅i̅v̅a̅t̅i̅o̅n_191 (Activation) | (None, 64, 64, 192) | 0 | |
| batch_normalization_191[0][0] | | | |
| a̅c̅t̅i̅v̅a̅t̅i̅o̅n_194 (Activation) | (None, 64, 64, 256) | 0 | |
| batch_normalization_194[0][0] | | | |
| b̅l̅o̅c̅k̅8̅_̅8̅_mixed (Concatenate) | (None, 64, 64, 448) | 0 | activation_191[0][0] |
| | | | activation_194[0][0] |
| b̅l̅o̅c̅k̅8̅_̅8̅_conv (Conv2D) | (None, 64, 64, 2080) | 933920 | block8_8_mixed[0][0] |
| b̅l̅o̅c̅k̅8̅_̅8̅ (Lambda) | (None, 64, 64, 2080) | 0 | block8_7_ac[0][0] |
| | | | block8_8_conv[0][0] |
| b̅l̅o̅c̅k̅8̅_̅8̅_ac (Activation) | (None, 64, 64, 2080) | 0 | block8_8[0][0] |
| c̅o̅n̅v̅2̅d̅_̅1̅9̅6 (Conv2D) | (None, 64, 64, 192) | 399360 | block8_8_ac[0][0] |
| b̅a̅t̅c̅h̅_̅n̅o̅r̅malization_196 (BatchN | (None, 64, 64, 192) | 576 | conv2d_196[0][0] |
| a̅c̅t̅i̅v̅a̅t̅i̅o̅n_196 (Activation) | (None, 64, 64, 192) | 0 | |
| batch_normalization_196[0][0] | | | |
| c̅o̅n̅v̅2̅d̅_̅1̅9̅7 (Conv2D) | (None, 64, 64, 224) | 129024 | activation_196[0][0] |
| b̅a̅t̅c̅h̅_̅n̅o̅r̅malization_197 (BatchN | (None, 64, 64, 224) | 672 | conv2d_197[0][0] |
| a̅c̅t̅i̅v̅a̅t̅i̅o̅n_197 (Activation) | (None, 64, 64, 224) | 0 | |
| batch_normalization_197[0][0] | | | |
| c̅o̅n̅v̅2̅d̅_̅1̅9̅5 (Conv2D) | (None, 64, 64, 192) | 399360 | block8_8_ac[0][0] |
| c̅o̅n̅v̅2̅d̅_̅1̅9̅8 (Conv2D) | (None, 64, 64, 256) | 172032 | activation_197[0][0] |
| b̅a̅t̅c̅h̅_̅n̅o̅r̅malization_195 (BatchN | (None, 64, 64, 192) | 576 | conv2d_195[0][0] |
| b̅a̅t̅c̅h̅_̅n̅o̅r̅malization_198 (BatchN | (None, 64, 64, 256) | 768 | conv2d_198[0][0] |
| a̅c̅t̅i̅v̅a̅t̅i̅o̅n_195 (Activation) | (None, 64, 64, 192) | 0 | |
| b̅a̅t̅c̅h̅_̅n̅o̅r̅malization_195[0][0] | | | |
| a̅c̅t̅i̅v̅a̅t̅i̅o̅n_198 (Activation) | (None, 64, 64, 256) | 0 | |
| batch_normalization_198[0][0] | | | |
| b̅l̅o̅c̅k̅8̅_̅9̅_mixed (Concatenate) | (None, 64, 64, 448) | 0 | activation_195[0][0] |
| | | | activation_198[0][0] |
| b̅l̅o̅c̅k̅8̅_̅9̅_conv (Conv2D) | (None, 64, 64, 2080) | 933920 | block8_9_mixed[0][0] |
| b̅l̅o̅c̅k̅8̅_̅9̅ (Lambda) | (None, 64, 64, 2080) | 0 | block8_8_ac[0][0] |
| | | | block8_9_conv[0][0] |
| b̅l̅o̅c̅k̅8̅_̅9̅_ac (Activation) | (None, 64, 64, 2080) | 0 | block8_9[0][0] |
| c̅o̅n̅v̅2̅d̅_̅2̅0̅0 (Conv2D) | (None, 64, 64, 192) | 399360 | block8_9_ac[0][0] |
| b̅a̅t̅c̅h̅_̅n̅o̅r̅malization_200 (BatchN | (None, 64, 64, 192) | 576 | conv2d_200[0][0] |
| a̅c̅t̅i̅v̅a̅t̅i̅o̅n_200 (Activation) | (None, 64, 64, 192) | 0 | |
| batch_normalization_200[0][0] | | | |
| c̅o̅n̅v̅2̅d̅_̅2̅0̅1 (Conv2D) | (None, 64, 64, 224) | 129024 | activation_200[0][0] |
| b̅a̅t̅c̅h̅_̅n̅o̅r̅malization_201 (BatchN | (None, 64, 64, 224) | 672 | conv2d_201[0][0] |
| a̅c̅t̅i̅v̅a̅t̅i̅o̅n_201 (Activation) | (None, 64, 64, 224) | 0 | |
| batch_normalization_201[0][0] | | | |
| c̅o̅n̅v̅2̅d̅_̅1̅9̅9 (Conv2D) | (None, 64, 64, 192) | 399360 | block8_9_ac[0][0] |
| c̅o̅n̅v̅2̅d̅_̅2̅0̅2 (Conv2D) | (None, 64, 64, 256) | 172032 | activation_201[0][0] |
| b̅a̅t̅c̅h̅_̅n̅o̅r̅malization_199 (BatchN | (None, 64, 64, 192) | 576 | conv2d_199[0][0] |
| b̅a̅t̅c̅h̅_̅n̅o̅r̅malization_202 (BatchN | (None, 64, 64, 256) | 768 | conv2d_202[0][0] |
| a̅c̅t̅i̅v̅a̅t̅i̅o̅n_199 (Activation) | (None, 64, 64, 192) | 0 | |
| batch_normalization_199[0][0] | | | |
| a̅c̅t̅i̅v̅a̅t̅i̅o̅n_202 (Activation) | (None, 64, 64, 256) | 0 | |
| batch_normalization_202[0][0] | | | |
| b̅l̅o̅c̅k̅8̅_̅1̅0̅_mixed (Concatenate) | (None, 64, 64, 448) | 0 | activation_199[0][0] |
| | | | activation_202[0][0] |
| b̅l̅o̅c̅k̅8̅_̅1̅0̅_conv (Conv2D) | (None, 64, 64, 2080) | 933920 | block8_10_mixed[0][0] |
| b̅l̅o̅c̅k̅8̅_̅1̅0̅ (Lambda) | (None, 64, 64, 2080) | 0 | block8_9_ac[0][0] |
| | | | block8_10_conv[0][0] |
| c̅o̅n̅v̅_̅7̅b̅ (Conv2D) | (None, 64, 64, 1536) | 3194880 | block8_10[0][0] |
| c̅o̅n̅v̅_̅7̅b̅_̅b̅n (BatchNormalization) | (None, 64, 64, 1536) | 4608 | conv_7b[0][0] |
| c̅o̅n̅v̅_̅7̅b̅_̅a̅c (Activation) | (None, 64, 64, 1536) | 0 | conv_7b_bn[0][0] |

| | | | |
|---|---|---|---|
| Total params: 54,336,736 | | | |

## Claims

1. A computer-implemented method for predicting a condition of health of a patient to be examined from a mass spectrometry, MS, specimen image representing a proteome of said patient, wherein x and y coordinates of a picture element, pixel, of the image indicate mass and elution time, also known as retention time, of a molecule in the proteome, and a value of the pixel indicates an abundance of the molecule in the proteome, the method comprising:
first training a neural network, NN, via an input layer, with a first plurality of sample MS images, also referred to as MS1 images, each sample image representing a proteome fulfilling a known first condition from a set of known conditions, wherein the first condition of each sample image in the first plurality of sample MS1 images is a binary or multiclass condition, designating features distinguishing sample images within the first plurality of sample images,
subsequent second training the NN, via the input layer, with a second plurality of sample MS1 images from a plurality of patients, each sample image representing a proteome fulfilling a known second binary or multiclass condition of health from a plurality of patient conditions,
wherein after this first training, the output layer of the trained NN is removed and replaced by an untrained output layer that is trained with the images of the second training round,;
obtaining, after the second training of the NN, in an input layer of the NN, a mass spectrometry, MS, image, representing a proteome expressing a condition of health of a patient to be examined;
forward propagating the MS image through the NN;
generating, via an output layer of the NN, a prediction of a condition of health of the patient to be examined.

2. The method of claim 1, wherein said distinguishing features learned in the first training of the neural network, NN, increase precision in subsequent second training of the NN with features similarly distinguishing sample MS1 images within the second plurality of sample images.

3. The method of claim 1 or 2, wherein the first condition of each sample image in the first plurality of sample MS1 images is a binary condition designating SILAC-labelled and label-free MS1 images, respectively, and wherein the second condition of each sample MS1 image in the second plurality of sample images from the plurality of patients, represents a binary condition of diseased or healthy, respectively, of said patient to be examined.

4. The method of any of the preceding claims, further comprising, after generating a prediction of a condition of the patient, identifying by a known method of Saliency Map explanation, the features contributing to the prediction of the condition of health of a patient to be examined; and
to provide a coarse localization heatmap of said features on top of the specimen image.

5. The method of any one of the preceding claims, wherein the neural network has one or more convolution layers.

6. The method of any one of the preceding claims, wherein the proteome comprises, preferably consists of histones.

7. A computer program product adapted to carry out the method as claimed in any one of the preceding claims.

## Patentansprüche

1. Computergestütztes Verfahren zur Vorhersage des Gesundheitszustands eines zu untersuchenden Patienten anhand eines Massenspektrometrie, MS, Probenbildes, das das Proteom des Patienten darstellt, wobei x- und y-Koordinaten eines Bildelements, Pixels, des Bildes die Masse und die Elutionszeit, auch als Retentionszeit bezeichnet, eines Moleküls im Proteom anzeigen und der Wert des Pixels die Häufigkeit des Moleküls im Proteom angibt, wobei das Verfahren umfasst:
erstes Training eines neuronales Netzwerks, NN, über eine Eingabeschicht, mit einer ersten Vielzahl von MS-Beispielbildern, auch als MS1-Bilder bezeichnet, wobei jedes Beispielbild ein Proteom darstellt, das einen bekannten ersten Zustand aus einer Reihe bekannter Zustände erfüllt, wobei der erste Zustand jedes Beispielbildes in der ersten Vielzahl von MS1-Beispielbildern ein binärer oder mehrklassiger Zustand ist, welcher Merkmale kennzeichnet, die die Beispielbilder innerhalb der ersten Vielzahl von Beispielbildern voneinander unterscheiden,
anschließendes zweites Training des NN über die Eingabeschicht, mit einer zweiten Vielzahl von MS1-Beispielbildern von einer Vielzahl von Patienten, wobei jedes Beispielbild ein Proteom darstellt, das einen bekannten zweiten binären oder mehrklassigen Gesundheitszustand aus einer Vielzahl von Patientenzuständen erfüllt,
wobei nach diesem ersten Training die Ausgabeschicht des trainierten NN entfernt und durch eine untrainierte Ausgabeschicht ersetzt wird, die mit den Bildern der zweiten Trainingsrunde trainiert wird,;
Erhalten eines Massenspektrometrie-Bildes, MS Bildes, in einer Eingabeschicht des NN nach dessen zweitem Training, wobei dieses Bild ein Proteom darstellt, das den Gesundheitszustand eines zu untersuchenden Patienten widerspiegelt;
Weiterleiten des MS-Bildes durch das NN;
Erzeugen einer Vorhersage über den Gesundheitszustand des zu untersuchenden Patienten über eine Ausgabeschicht des NN.

2. Verfahren nach Anspruch 1, wobei die bei dem ersten Training des neuronalen Netzes, NN, erlernten Unterscheidungsmerkmale die Genauigkeit im anschließenden zweiten Training des NN mit Merkmalen, die in ähnlicher Weise MS1 Beispielbilder innerhalb der zweiten Vielzahl von Beispielbildern unterscheiden, erhöhen.

3. Verfahren nach Anspruch 1 oder 2, wobei der erste Zustand jedes Beispielbildes in der ersten Vielzahl von MS1-Beispielbildern ein binärer Zustand ist, der SILACmarkierte bzw. markierungsfreie MS1-Bilder bezeichnet, und wobei der zweite Zustand jedes MS1-Beispielbildes in der zweiten Vielzahl von Beispielbildern aus der Vielzahl von Patienten einen binären Zustand von Krankheit bzw. Gesundheit des zu untersuchenden Patienten darstellt.

4. Verfahren nach einem der vorhergehenden Ansprüche, welches ferner umfasst, dass nach dem Erzeugen einer Vorhersage über den Zustands des Patienten mittels eines bekannten Verfahrens zur Saliency-Map-Erklärung die Merkmale identifiziert werden, die zur Vorhersage des Gesundheitszustands eines zu untersuchenden Patienten beitragen; und
Bereitstellen einer groben Lokalisierungs-Heatmap dieser Merkmale über dem Probenbild.

5. Verfahren nach einem der vorhergehenden Ansprüche, wobei das neuronale Netzwerk eine oder mehrere Faltungsschichten aufweist.

6. Verfahren nach einem der vorhergehenden Ansprüche, wobei das Proteom Histone umfasst, vorzugsweise aus Histonen besteht.

7. Computerprogrammprodukt, das zur Durchführung des in einem der vorstehenden Ansprüche beanspruchten Verfahrens angepasst ist.

## Revendications

1. Procédé mis en œuvre par ordinateur pour prédire l'état de santé d'un patient à examiner à partir d'une image d'échantillon obtenue par spectrométrie de masse, MS, représentant le protéome dudit patient, dans lequel les coordonnées x et y d'un pixel de l'image indiquent la masse et le temps d'élution, également appelé temps de rétention, d'une molécule dans le protéome, et la valeur du pixel indique l'abondance de la molécule dans le protéome, le procédé comprenant :
premier apprentissage d'un réseau neuronal, NN, via une couche d'entrée, à l'aide d'un premier ensemble d'images MS d'échantillons, également appelées images MS1, chaque image d'échantillon représentant un protéome répondant à une première condition connue parmi un ensemble de conditions connues, la première condition de chaque image d'échantillon du premier ensemble d'images MS1 étant une condition binaire ou multiclasses, désignant des caractéristiques permettant de distinguer les images d'échantillons au sein du premier ensemble d'images d'échantillons,
ensuite, entraîner à nouveau le réseau neuronal, via la couche d'entrée, à l'aide d'un deuxième ensemble d'images MS1 provenant d'un ensemble de patients, chaque image représentant un protéome répondant à une deuxième condition binaire ou multiclasses connue de l'état de santé parmi un ensemble d'états de santé des patients,
dans lequel, après ce premier apprentissage, la couche de sortie du réseau neuronal entraîné est supprimée et remplacée par une couche de sortie non entraînée qui est entraînée à l'aide des images du deuxième cycle d'apprentissage,;
obtenir, après le deuxième apprentissage du NN, dans une couche d'entrée de ce réseau, une image de spectrométrie de masse, MS, représentant un protéome reflétant l'état de santé d'un patient à examiner;
en faisant passer l'image du modèle de base MS à travers le NN;
générer, via une couche de sortie du NN, une prédiction de l'état de santé du patient à examiner.

2. Procédé selon la revendication 1, dans lequel lesdites caractéristiques distinctives apprises lors du premier apprentissage du réseau neuronal, NN, améliorent la précision lors d'un deuxième apprentissage ultérieur du NN avec des caractéristiques permettant de distinguer de manière similaire des images d'échantillon MS1 au sein de la deuxième pluralité d'images d'échantillon.

3. Procédé selon la revendication 1 ou 2, dans lequel la première condition de chaque image échantillon de la première série d'images MS1 échantillons est une condition binaire désignant respectivement des images MS1 marquées par SILAC et des images MS1 non marquées, et dans lequel la deuxième condition de chaque image MS1 échantillon de la deuxième série d'images échantillons provenant de la pluralité de patients représente une condition binaire indiquant respectivement si ledit patient à examiner est malade ou en bonne santé.

4. Procédé selon l'une quelconque des revendications précédentes, comprenant en outre, après avoir généré une prédiction de l'état de santé du patient, l'identification, à l'aide d'une méthode connue d'explication par carte de saillance, des caractéristiques contribuant à la prédiction de l'état de santé d'un patient à examiner; et
la fourniture d'une carte thermique de localisation approximative desdites caractéristiques superposée à l'image de l'échantillon.

5. Procédé selon l'une quelconque des revendications précédentes, dans lequel le réseau neuronal comporte une ou plusieurs couches de convolution.

6. Procédé selon l'une quelconque des revendications précédentes, dans lequel le protéome comprend, de préférence se compose d'histones.

7. Un produit logiciel adapté pour mettre en œuvre le procédé selon l'une quelconque des revendications précédentes.
